# EUROPEAN PATENT APPLICATION

(11) **EP 1 645 556 A1**
(43) Date of publication of application: **12.04.2006**
(21) Application number: 04023926.1
(22) Date of filing: 07.10.2004
(51) Int. Cl.: C07D 295/18, C07D 213/74, C07D 207/32, C07D 317/46, C07D 231/12, C07D 213/56, C07D 213/64, A61K 31/495, A61P 35/00

(54) **Arylpiperazine-benzoylamide derivatives useful as pharmaceutical agents**

(71) Applicant: Boehringer Ingelheim International GmbH, 55216 Ingelheim (DE)
(72) Inventor: Betzemeier, Bodo, A-1140 Wien (AT); Krist, Bernd, A-1040 Vienna (AT); McConnell, Darryl, A-1200 Vienna (AT); Steurer, Steffen, A-1190 Vienna (AT); Impagnatiello, Maria, A-1030 Vienna (AT); Weyer-Czernilofsky, Ulrike, A-2500 Baden (AT); Hilberg, Frank, A-1050 Vienna (AT); Brueckner, Ralph, A-1130 Vienna (AT); Daiimann, Georg, D-88448 Attenweiler (DE); Heckel, Armin, D-88400 Biberach (DE); Kley, Joerg, D-88441 Mittelbiberach (DE); Lehmann-Lintz, Thorsten, D-88416 Ochsenhausen (DE); Roth, Gerald, D-88400 Biberach (DE)
(74) Representative: Hammann, Heinz

(57) **Abstract**

The invention relates to arylpiperazine-benzoylamide derivatives of formula (I) 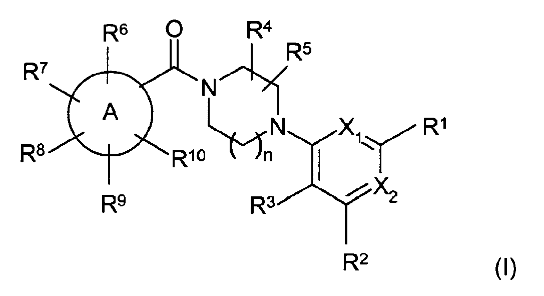 wherein A, X₁, X₂, n and R¹ to R¹⁰ are defined as in claim 1, which are suitable for the treatment of diseases characterized by excessive or abnormal cell proliferation and the use thereof for preparing a pharmaceutical composition.

## Description

### SUMMARY OF THE INVENTION

The present invention relates to arylpiperazine-benzoylamide derivatives of formula (I) 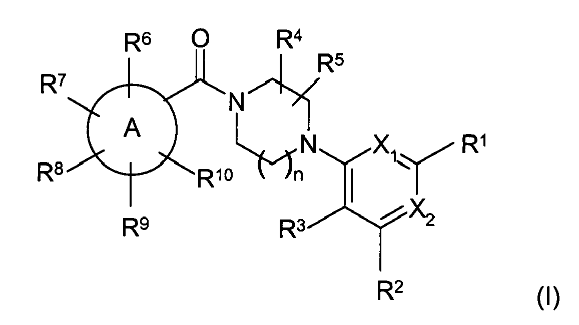 to their tautomers, enantiomers, diastereomers, mixtures thereof and their physiologically acceptable salts which have useful pharmacological properties, to processes for preparing them and medicaments comprising them and to the use.

### BACKGROUND OF THE INVENTION

Microtubules are cytoskeletal structures assembled from α/β tubulin heterodimers that play an essential role in many cellular processes, such as cell motility, organelle transport, maintenance of cell polarity and cell division. Interference with microtubule dynamics by stabilization or destabilization in dividing cells leads to cell division arrest in the G₂/M phase and cell death.

A variety of clinically promising compounds which demonstrate potent cytotoxicity and antitumor activity are known to effect their primary mode of action through an efficient inhibition of tubulin. Several natural products and their derivatives disrupt microtubule dynamics, e.g., Taxol®, Taxotere®, Navelbine® and show a clinically useful therapeutic window between anticancer effects and dose-limiting toxicity in normal proliferating tissues, notably bone marrow and gastrointestinal mucosa in addition to neurotoxicity. Unfortunately the clinical success of these agents can be severely hindered by the emergence of drug resistant tumor cells. Although membrane P-glycoprotein mediated multi-drug resistance (MDR) has been known to occur with the taxanes and the *Vinca* alkaloids, differential expression of altered tubulin isotypes has also been implicated in resistance to the taxanes and other antimitotic agents.
Renewed interest in tubulin polymerization inhibitors has been generated by the hope that non-MDR substrates that interact with tubulin at sites near to, overlapping with or different from those of the taxanes or the *Vinca* alkaloids can be discovered.

Novel tubulin-binding molecules which, upon binding to tubulin, interfere with tubulin polymerization can provide novel agents for the treatment of cancer.

Piperazine derivatives are described as useful calmodolin inhibitors (EP 0 624 584 A1) for treating a circulatory disease or a disease in the cerebral region.

WO 9837077A1 discloses piperazine compounds substituted in 1-position with phenylamide or a benzyl group and in 4-position with a benzyl group as calcitonin mimetics useful for treating hypercholesterolemia and also as anti-fungal agents. Piperazine derivatives (WO 9728128) are also described for the treatment of hypercholesterolemia and as anti-fungal agents by inhibiting the enzyme oxido squalene cyclase.

Heteroaryl substituted piperazine derivatives are described as capsaicin receptor ligands in the treatment of chronic and acute pain conditions and urinary incontinence (WO 0208221A2).

### DETAILED DESCRIPTION OF THE INVENTION

Surprisingly it was found that compounds of the formula (I), wherein the residues A, R¹⁻R¹⁰, n, X₁ and X₂ have the meaning as defined herein, act as tubulin polymerisation inhibitors.

The invention therefore relates to a compound of formula (I) 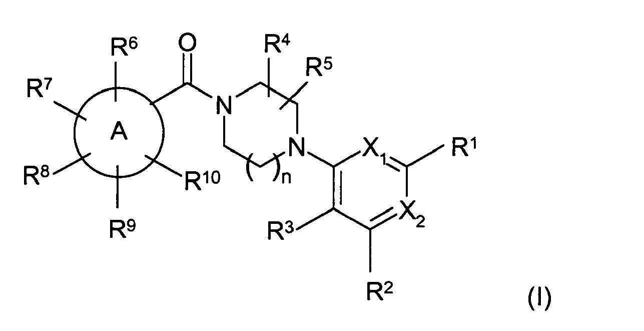 its salts or pharmaceutically acceptable derivatives thereof, wherein
A represents mono- or bicyclic carbocyclic aryl; and
**R**^{**1**} and **R**^{**2**} are independently selected from the group consisting of hydrogen, halogen and cyano or from an optionally substituted group consisting of C₁₋₆alkyl-, R^{a}O-, R^{a}R^{b}N-, C₁₋₆alkyl-C(=O)-, C₁₋₆alkyl-C(=O)-N(R^{b})-, C₁₋₆alkyl-N(R^{b})-C(=O)- and R^{a}O-C₁₋₆alkyl-, and
**R**^{**3**} is selected from the group consisting of hydrogen, halogen, cyano, C₁₋₆alkyl- and R^{a}O-or
**R**^{**2**} and **R**^{**3**} may combine to form an optionally substituted cyclic group -O-(CH₂)ₚ-O-; and
**R**^{**4**} and **R**^{**5**} are independently selected from hydrogen and C₁₋₆alkyl-; and
**R**^{**6**}**, R**^{**7**}, **R**^{**8**}, **R**^{**9**} and **R**^{**10**} are independently selected from the group consisting of hydrogen, halogen, nitro and cyano or from an optionally substituted group consisting of C₁₋₆alkyl-, R^{a}R^{b}N-, R^{a}O-, R^{a}-C(=O)-N(R^{b})-, R^{a}R^{b}N-C(=O)-, R^{a}R^{b}-SO₂-, R^{a}-SO₂-N(R^{b})-, R^{a}-C(=O)-, R^{a}-SO₂-, R^{a}-SO-, R^{a}-S-, R^{a}R^{b}N-C₁₋₆alkyl-, R^{a}R^{b}N-C₁₋₆alkyl-O-, R^{a}O-C₁₋₆alkyl- and aryl; and
**R**^{**a**} and **R**^{**b**} are independently selected from hydrogen, C₁₋₆alkyl-, cycloalkyl-, aryl-C₁₋₆alkyl- and aryl; and
**X**_{**1**} and **X**_{**2**} are independently selected from CH, CF or N; and
**n** is 1 or 2; and
**p** is 1 or 2.

A further aspect of the invention is a compound of formula (I) wherein
**X**_{**2**} represents CH or CF; and
n represents 1.

An additional aspect of the invention is a compound of formula (Ia), 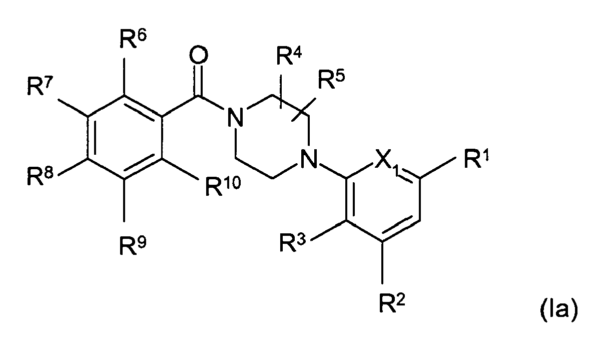 its salts or pharmaceutically acceptable derivatives thereof, wherein
**R**^{**1**} and **R**^{**2**} are independently selected from the group consisting of hydrogen, halogen and cyano or from an optionally substituted group consisting of C₁₋₆alkyl-, R^{a}O-, R^{a}R^{b}N-, C₁₋₆alkyl-C(=O)-, C₁₋₆alkyl-C(=O)-N(R^{b})-, C₁₋₆alkyl-N(R^{b})-C(=O)- and R^{a}O-C₁₋₆alkyl-; and
**R**^{**3**} is selected from the group consisting of hydrogen, halogen, cyano, C₁₋₆alkyl- and R^{a}O-, or
**R**² and **R**³ may combine to form an optionally substituted cyclic group -O-(CH₂)ₚ-O-; and
**R**⁴ and **R**⁵ are independently selected from hydrogen and C₁₋₆alkyl-; and
**R**⁶, **R**⁷, **R**⁸**, R**⁹ and **R**¹⁰ are independently selected from the group consisting of hydrogen, halogen, nitro and cyano or from an optionally substituted group consisting of C₁₋₆alkyl-, R^{a}R^{b}N-, R^{a}O-, R^{a}-C(=O)-, R^{a}-C(=O)-N(R^{b})-, R^{a}R^{b}N-C(=O)-, R^{a}R^{b}N-SO₂-, R^{a}-SO₂-N(R^{b})-, R^{a}-C(=O)-, R^{a}-SO₂-, R^{a}-SO-, R^{a}-S-, R^{a}R^{b}-C₁₋₆alkyl-, R^{a}R^{b}N-C₁₋₆akyl-O-, R^{a}O-C₁₋₆alkyl-and aryl; and
**R**^{**a**} and **R**^{**b**} are independently selected from hydrogen, C₁₋₆alkyl-, cycloalkyl-, aryl-C₁₋₆alkyl- and aryl; and
X₁ is selected from CH, CF or N; and
**p** is 1 or 2.

Another aspect of the invention is a compound of formula (Ia), wherein X₁ represents CH or CF.

Yet, another aspect of the invention is a compound of formula (Ia), wherein
**R**^{**6**}, **R**^{**7**}**, R**^{**8**}**, R**^{**9**} and **R**^{**10**} are independently selected from the group consisting of hydrogen, halogen, nitro and cyano or from an optionally substituted group consisting of C₁₋₆alkyl-, R^{a}R^{b}N-, R^{a}O-, R^{a}-C(=O)-, R^{a}R^{b}N-C₁₋₆alkyl-, R^{a}R^{b}N-C₁₋₆alkyl-O-, R^{a}O-C₁₋₆alkyl- and aryl.

One aspect of the invention is a compound of formula (I) or (Ia) as medicament.

Another aspect of the invention is a compound of formula (I) or (Ia) as antiproliferative medicament.

A further aspect of the invention is the use of a compound of formula (I) or (Ia) for the manufacture of a medicament for the treatment of a proliferative disease.

An alternative aspect of the invention is the use of a compound of formula (I) or (Ia) for the manufacture of a medicament for the treatment of cancer.

An additional aspect of the invention is the use of a compound of formula (I) or (Ia) for the manufacture of a medicament for the treatment of conditions ameliorated by an inhibitory action on tubulin polymerization

One aspect of the invention is also pharmaceutical composition containing as active ingredient one or more compounds of formula (I) or (Ia) or their physiologically acceptable salts in combination with a usual adjuvants and/or carrier.

Yet, another aspect of the invention is a pharmaceutical composition comprising a compound of formula (I) 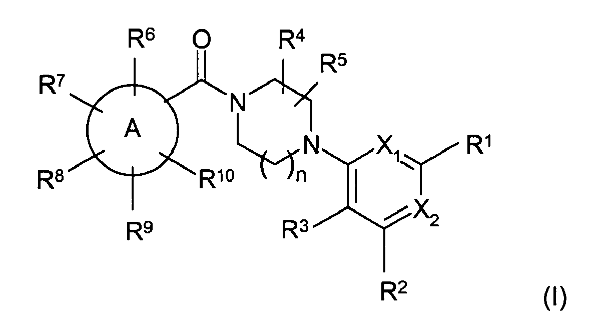 its salts or pharmaceutically acceptable derivatives thereof, wherein

A represents mono- or a bicyclic carbocyclic aryl; and
**R**^{**1**} and **R**² are independently selected from the group consisting of hydrogen, halogen and cyano or from an optionally substituted group consisting of C₁₋₆alkyl-, R^{a}O-, R^{a}R^{b}N-, C₁₋₆alkyl-C(=O)-, C₁₋₆alkyl-C(=O)-N(R^{b})-, C₁₋₆alkyl-N(R^{b})-C(=O)- and R^{a}O-C₁₋₆alkyl-, and
**R**^{**3**} is selected from the group consisting of hydrogen, halogen, cyano, C₁₋₆alkyl- and R^{a}O-, or
**R**^{**2**} and **R**^{**3**} may combine to form an optionally substituted cyclic group -O-(CH₂)ₚ-O-; and
**R**^{**4**} and **R**^{**5**} are independently selected from hydrogen and C₁₋₆alkyl-; and
**R**^{**6**}**, R**^{**7**}, **R**^{**8**}**, R**^{**9**} and **R**^{**10**} are independently selected from the group consisting of hydrogen, halogen, nitro, cyano or from an optionally substituted group consisting of C₁₋₆alkyl-, R^{a}R^{b}N-, R^{a}O-, R^{a}-C(=O)-N(R^{b})-, R^{a}R^{b}N-C(=O)-, R^{a}R^{b}N-SO₂-, R^{a}-SO₂-N(R^{b})-, R^{a}-C(=O)-, R^{a}-SO₂-, R^{a}-SO-, R^{a}-S-, R^{a}R^{b}-C₁₋₆alkyl-, R^{a}R^{b}N-C₁₋₆alkyl-O-, R^{a}O-C₁₋₆alkyl-, and aryl; and
**R**^{**a**} and **R**^{**b**} are independently selected from hydrogen, C₁₋₆alkyl-, cycloalkyl-, aryl-C₁₋₆alkyl- and aryl; and
**X**_{**1**} and **X**_{**2**} are independently selected from CH, CF or N; and
**n** is 1 or 2; and
**p** is 1 or 2
or a tautomer, a pharmaceutically acceptable salt, solvate, or physiologically functional derivative thereof, and
at least one different cytostatic and/or cytotoxic active ingredient or a pharmaceutically acceptable salt, solvate, or physiologically functional derivative thereof, and
a pharmaceutically acceptable carrier or excipient.

### DEFINITIONS

As used herein, the following definitions shall apply unless otherwise indicated.

The term "optionally substituted" is used interchangeably with the phrase "substituted or unsubstituted" and means that a group may be substituted by one or more substituents which may be the same or different. When otherwise not specified these substituents are selected from alkyl, cycloalkyl, biaryl, carbocyclic aryl, heteroalicyclic, heteroaryl, acyl, amidino, amido, amino, alkoxyamino, carbamoyl, carboxy, cyano, ether, guanidine, hydroxamoyl, hydroxyl, imino, isocyanato, isothiocyanato, halo, nitro, silyl, sulfonyl, sulfinyl, sulfenyl, sulfonato, sulfamoyl, sulfonamido, thiocarbonyl, thiol, thiocyanato, thiocarbamoyl, thioamido or urea as those terms are define herein.

As used herein, the term "alkyl" refers to an aliphatic hydrocarbon group. The alkyl moiety may be a "saturated alkyl" group, which means that it does not contain any alkene or alkyne moieties. The alkyl moiety may also be an "unsaturated alkyl" moiety, which means that it contains at least one alkene or alkyne moiety. An "alkene" moiety refers to a group consisting of at least two carbon atoms and at least one carbon-carbon double bond. An "alkyne" moiety refers to a group consisting of at least two carbon atoms and at least one carbon-carbon triple bond. The alkyl moiety, whether saturated or unsaturated, may be branched or non-branched. Branched means that the alkyl moiety is substituted by one or more lower alkyl groups such as for example methyl, ethyl or propyl.
The alkyl group may have the number of carbon atoms as explicitly defined (e.g.
C₁₋₁₂alkyl) or may also be undefined. Whenever it appears herein a numerical range such as "1 to 12" it refers to each integer in the given range. For example, "1 to 12 carbon atoms" means that the alkyl group may consist of 1 carbon atom, 2 carbon atoms, 3 carbon atoms, etc., up to and including 12 carbon atoms. When the number of carbon atoms is undefined the alkyl group has 1 to 12 carbon atoms. A medium sized alkyl refers to an alkyl group having 1 to 8 carbon atoms. A lower alkyl group refers to an alkyl group having 1 to 5 carbon atoms. The alkyl group, whether termed an alkyl, saturated alkyl, unsaturated alkyl, alkene or alkyne, may be unsubstituted or substituted as defined herein. One or more hydrogens of an alkyl group may be replaced by a moiety selected from the group consisting of hydroxy, halo, cyano, amino, thiol, C₁₋₆alkoxy, C₁₋₆alkylthio, C₁₋₆alkylamino and C₁₋₆dialkylamino.

The term "cycloalkyl" as used herein refers to mono- or bicyclic ring or ring systems consisting of 3 to 11 carbon atoms. The ring system may be a "saturated ring", which means that the ring does not contain any alkene or alkyne moieties. The cycloalkyl group may also be an "unsaturated ring" which means that it contains at least one alkene or alkyne moiety and provided that the ring system is not aromatic. The cycloalkyl group may be unsubstituted or substituted as defined herein. In addition to the above mentioned substituents one or more ring carbon atoms may also be bonded via a double bond to a heteroatom selected from N, S and O. The cycloalkyl substituent may be bonded via a linker group such as a lower alkyl group, or by any ring atom. One or more hydrogens of an alkyl group may be replaced by a moiety selected from the group consisting of hydroxy, halo, cyano, amino, thiol, C₁₋₆alkoxy, C₁₋₆alkylthio, C₁₋₆alkylamino and C₁₋₆dialkylamino.

The term "aryl" as used herein refers to a mono- or bicyclic ring or ring systems which have at least one aromatic ring. Aryl groups include both, "carbocyclic aryl" and "heteroaryl" groups as defined herein. The aryl moiety may be unsubstituted or substituted as defined herein and the substituents, when positioned adjacent to one another, may combine to form cycloalkyl or heteroalicyclic ring systems for example methylendioxy or difluoromethylendioxy. An aryl substituent is linked via an aromatic ring system.

The term "carbocyclic" refers to a compound which contains one or more covalently closed ring structures and the atoms forming the backbone of the ring(s) are all carbon atoms. The term thus distinguishes carbocyclic from heterocyclic rings. Carbocyclic groups include both, a "cycloalkyl group", which means a non-aromatic carbocycle, and a "carbocyclic aryl" group, which means an aromatic carbocycle. The carbocyclic group may optionally be substituted as defined herein.

The term "carbocyclic aryl" as used herein refers to mono- or bicyclic rings or ring systems which have at least one aromatic ring and all atoms forming the backbone are carbon atoms. Examples of carbocyclic aryl groups include but are not limited to phenyl, and naphthyl. The carbocyclic aryl moiety may be unsubstituted or substituted as defined herein and the substituents, when positioned adjacent to one another, may combine to form cycloalkyl or heteroalicyclic ring systems for example methylendioxy or difluoromethylendioxy.

The term "heterocyclic" or "heterocyclo" as used herein refers to mono- or bicyclic rings or ring systems which include one or more heteroatoms selected from N, S and O. The rings or ring systems include 1 to 13 carbon atoms in addition to the heteroatom(s). The term heterocyclic group include both, a "heteroalicyclic" group, which means a non-aromatic heterocycle and a "heteroaryl" group, which means an aromatic heterocycle. The heterocyclic moiety may be unsubstituted or substituted as defined herein and the substituents, when positioned adjacent to one another, may combine to form cycloalkyl or heteroalicyclic ring systems for example methylendioxy or difluoromethylendioxy. The heterocyclic group may be bonded via a carbon atom or a heteroatom. The heterocyclic group may also include the oxides of nitrogen and sulfur if nitrogen or sulfur are present in the ring.

The term "heteroalicyclic" or "heteroalicyclo" as used herein refers to mono- or bicyclic ring or ring systems in which at least one of the atoms forming the backbone of the ring is a heteroatom. The ring system may be a "saturated ring", which means that the ring does not contain any alkene or alkyne moieties, or it may also be an "unsaturated ring" which means that it contains at least one alkene or alkyne moiety provided that the ring system is not aromatic. The heteroalicyclic group may be unsubstituted or substituted as defined herein. The substituents, when positioned adjacent to one another, may combine to form carbocyclic or heterocyclic ring systems for example methylendioxy or difluoromethylendioxy. The heteroalicyclic group may be bonded via a carbon atom or a heteroatom. In addition to the above mentioned substituents one or more ring carbon atoms may also be bonded via a double bond to a heteroatom selected from N, S and O. The heteroalicyclic group may also include the oxides of nitrogen and sulfur if nitrogen or sulfur are present in the ring.

The term "heteroaryl", "heterocyclic aryl" or "heteroaromatic radical" as used herein refers to a mono- or bicyclic rings or ring systems which include one or more heteroatoms selected from N, S and O. The rings or ring systems include 1 to 13 carbon atoms in addition to the heteroatom(s) and contains at least one aromatic ring with a heteroatom. The heteroaryl group may also include the oxides of nitrogen and sulfur if nitrogen or sulfur is present, or an oxo-group respectively. Examples of monocyclic heteroaryl groups include but are not limited to furyl, thienyl, pyrrolyl, oxazolyl, thiazolyl, isoxazolyl, isothiazolyl, pyrazolyl, imidazolyl, triazolyl, tetrazolyl, oxadiazolyl, thiadiazolyl, pyridyl, pyrimidyl, pyridazinyl, pyrazinyl and triazinyl. Examples of bicyclic heterocycles include but are not limited to indolyl, benzofuranyl, benzothienyl, benzoxazolyl, benzothiazolyl, benzisoxazolyl, benzisothiazolyl, indazolyl, isoquinolinyl, quinolinyl, quinoxalinyl, cinnolinyl, phthalazinyl, quinazolinyl, benzotriazinyl and the like. Examples of tricyclic heterocycles include but are not limited to thianthrenyl, xanthenyl, phenoxathiinyl, carbazolyl, carbolinyl, phenanthridinyl, acridinyl, perimidinyl, phenanthrolinyl, phenazinyl, phenothiazinyl, phenoxazinyl. The heteroaryl group may be unsubstituted or substituted as defined herein. The substituents, when positioned adjacent to one another, may combine to form a cycloalkyl or heteroalicyclic ring for example methylendioxy and difluoromethylendioxy. The heteroaryl radical may be bonded via a carbon atom or a heteroatom.

The term "carbocyclic arylalkyl", as used herein, refers to a chemical moiety of formula carbocyclic aryl-C₁₋₆alkyl- as those terms are defined herein.

The term "acyl", as used herein, refers to a chemical moiety of formula ―(CH₂)ₓC(=O)R^{z}.

The term "amidino" refers to a chemical moiety with the formula ―(CH₂)ₓC(=NH)NR^{z}R'^{z}.

The term "amido" refers to both, a "C-amido" group which means a chemical moiety with the formula -(CH₂)ₓC(=O)NR^{z}R^{'z} and a "N-amido" group which means a chemical moiety with the formula -(CH₂)ₓNR^{z}C(=O)R^{'z}.

The term "amine" or "amino" refers to a chemical moiety of formula ―(CH₂)ₓNR^{z}R'^{z}. The definition of an amine is also understood to include their N-oxides.

The term "alkoxyamino", refers to both, an "N-alkoxyamino" group which means a chemical moiety with the formula ―(CH₂)ₓNR^{z}OR'^{z} and an "O-alkoxyamino" group which means a chemical moiety with the formula ―(CH₂)ₓONR^{z}R'^{z}.

The term "carbamoyl" refers to both, an "O-carbamoyl" group which means a chemical moiety with the formula ―(CH₂)ₓOC(=O)NR^{z}R'^{z} and a "N-carbamoyl" group which means a chemical moiety with the formula ―(CH₂)ₓNR^{z}C(=O)OR'^{z}.

The term "carboxy" refers to both, an "O-carboxy" group which means a chemical moiety with the formula-(CH₂)ₓOC(=O)R^{z} and a "C-carboxy" group which means a chemical moiety with the formula ―(CH₂)ₓC(=O)OR^{z}.

A "cyano" group refers to a -(CH₂)ₓC≡N.

The term "hydroxamoyl" refers to a chemical moiety with the formula -(CH₂)ₓC(=O)NOR^{z}.

The term "hydroxy" or "hydroxyl" as used herein, refers to a chemical moiety of formula -OH.

The term "imine" or "imino", as used herein, refers to a chemical moiety of formula -(CH₂)ₓ(R^{z})C=NR'^{z}.

The term "halogen" or "halo" refers to an atom selected from the group consisting of fluorine, chlorine, bromine and iodine.

The term "carbonyl", as used herein, refers to to a chemical moiety with the formula - -(CH₂)ₓC(=O)R^{z}.

The term "sulfone" or "sulfonyl" refers to a chemical moiety with the formula -(CH₂)ₓS(=O)₂R^{z}.

The term "sulfinyl" refers to a chemical moiety with the formula -(CH₂)ₓS(=O)R^{z}.

The term "sulfenyl" refers to a chemical moiety with the formula -(CH₂)ₓSR^{z}.

The term "sulfonato" refers to both, an "S-sulfonato" group which means a chemical moiety with the formula ―(CH₂)ₓS(=O)₂OR^{z} and an "O-sulfonato" group which means a chemical moiety with the formula ―CH₂)ₓOS(=O)₂R^{z}.

A "sulfamoyl" group refers to a chemical moiety with the formula -(CH₂)ₓNR''^{z}S(=O)₂NR^{z}R'^{z}.

The term "sulfonamido" refers to both, an "S-sulfonamido" group which means a chemical moiety with the formula ―(CH₂)ₓS(=O)₂NR^{z}R'^{z} and an "N-sulfonamido" group which means a chemical moiety with the formula -(CH₂)ₓNRₐS(=O)₂R'^{z}.

The term "thiocarbonyl" refers to a chemical moiety with the formula ―(CH₂)ₓC(=S)R^{z}.

The term "thio" or "thiol", as used herein, refers to a chemical moiety of formula -SH.

A "thiocyanato" group refers to a ―CNS group.

The term "thiocarbamoyl" refers to both, an "O-thiocarbamoyl" group which means a chemical moiety with the formula -(CH₂)ₓOC(=S)NR^{z}R'^{z} and a "N-thiocarbamoyl" group which means a chemical moiety with the formula -(CH₂)ₓNR^{z}C(=S)OR'^{z}.

The term "thioamide" refers to both, a "C-thioamido" group which means a chemical moiety with the formula ―(CH₂)ₓC(=S)NR^{z}R'^{z} and a "N-thioamido" group which means a chemical moiety with the formula ―(CH₂)ₓNR^{z}C(=S)R'^{z}.

An "urea" group refers to a -(CH₂)ₓNR"^{a}C(=O)NR^{z}R'^{z}.

The term "formyl", as used herein, refers to a chemical moiety of formula ―C(=O)H.

The term "oxime ether" as used herein, refers to a chemical moiety of formula -(CH₂)ₓ(R^{z})C=NOR^{z}.
x is 0, 1, 2, 3 or 4.
R^{z} and R'^{z} are independently selected from the group consisting of hydrogen, C₁₋₆alkyl-, cycloalkyl-, aryl-C₁₋₆alkyl- and aryl.

In order that this invention be more fully understood, the following examples are set forth. These examples are for the purpose of illustrating embodiments of this invention and are not to be construed as limiting the scope of the invention in any way.

The examples which follow are illustrative and, as recognized by one skilled in the art, particular reagents or conditions could be modified as needed for individual compounds. Starting materials used in the scheme below are either commercially available or easily prepared from commercially available materials by those skilled in the art.

### Description of the Examples

### General experimental description of examples

HPLC retention times and mass spectra are recorded according to methods AM1 to AM5. 1H NMR spectra are recorded with either NMR Avance 400 (400, 1330810 MHz) or NMR Avance 500 (500, 1300038 MHz). Microwave heating is performed with either a Personal Chemistry Smith Synthesizer or a CEM Explorer.

### When otherwise not mentioned "aqueous workup" refers to following procedure:

After completion of the reaction and removal of solvents the residue is taken up in H₂O and EtOAc. The aqueous phase is extracted with EtOAc (up to 3 times). The combined organic layer is washed with saturated NaCl solution, dried over MgSO₄ and evaporated.

### Intermediates and starting materials

The following starting materials are commercially available:
(I.1) 4-Amino-3,5-dichloro-benzoic acid
(1.2) 3,5-Dichloro-benzoic acid
(I.3) 3,5-Dichloro-4-hydroxy-benzoic acid
(I.4) 3-Chloro-4-hydroxy-5-methoxy-benzoic acid
(1.5) 2,3,5-Trichloro-benzoic acid
(1.6) 3-Hxdroxy-4-methyl-benzoic acid
(I.7) 2,4,5-Trimethoxy-benzoic acid
(1.8) 2-(1-Pyrrolyl)-benzoic acid
(1.9) 3,4-Dimethoxy-benzoic acid
(I.10) 1-Naphthoic acid
(I.11) 3-Methoxy-4-methyl-benzoic acid
(I.12) 3,5-Dimethyl-benzoic acid
(I.13) 3,5-Bis(trifluoromethyl)-benzoic acid
(I.14) 9-Anthracenecarboxylic acid
(I.15) 2,3,4-Trimethoxy-benzoic acid
(I.16) 2-Biphenylcarboxylic acid
(1.17) 3,4-Dichloro-benzoic acid
(I.18) 3-Bromo-4-methoxy-benzoic acid
(I.19) 3-Hydroxy-4-methoxy-benzoic acid
(1.20) 2-Chloro-3,4-dimethoxy-benzoic acid
(I.21) 4-Methoxy-3-methyl-benzoic acid
(1.22) 3-Amino-2,5-dichloro-benzoic acid
(1.23) 2-Iodo-benzoic acid
(1.24) 3,4,5-Trimethoxy-benzoic acid
(II.1) 1-(3-Chlorophenyl)-piperazine
(II.2) 1-(3-Trifluoromethylphenyl)-piperazine
(II.3) 1-(3-Methylphenyl)-piperazine
(II.4) 1-(3-Hydroxyphenyl)-piperazine
(II.5) 1-(2,3-Dichlorophenyl)-piperazine
(II.6) 1-(3-Methoxyphenyl)-piperazine
(II.7) 1-(3,5-Dichlorophenyl)-piperazine
(II.8) 1-(2-(6-Methylpyridyl))-piperazine
(II.9) 1-(3,5-Dimethoxyphenyl)-piperazine
(II.10) 1-[3-(Trifluoromethoxy)-phenyl]-piperazine
(II.11) 1-(2,5-Dimethoxyphenyl)-piperazine
(V.1) 3-Cyanophenyl-boronic acid
(V.2) 4-Methoxypheneyl-boronic acid
(V.3) 4-Dimethylaminophenyl-boronic acid
(V.4) Pyridine-4-boronic acid
(V.5) 3-Aminophenyl-boronic acid
(V.6) Pyridine-3-boronic acid
(V.7) 2-Methoxy-5-pyridine-boronic acid
(V.8) Pyrazole-3-boronic acid (purchased from ChemBridge)

The following intermediates are prepared as described in the literature:
(III.1) 4-Amino-3,5-dibromo-benzoic acid (Recl. Trav. Chim. Pays-Bas 43 (1924) 867)
(III.2) 3,5-Dichloro-4-methoxy-benzoic acid (J. Med. Chem. 39,1 (1996) 253-266)
(III.3) 4-Amino-3-chloro-5-trifluoromethyl-benzoic acid (Arzneim. Forsch. 34 (1984) 1612-1624)
(III.4) 5,6,7,8-Tetrahydro-1-napthalenecarboxylic acid (J. Org. Chem. 51, 26 (1986) 5452-5454)
(III.5) 3,4-Dimethoxy-5-methyl-benzoic acid (J. Med. Chem. 25,3 (1982) 263-271)
(III.6) 3-Methoxy-5-trifluoromethyl-benzoic acid (US 3953595)
(III.7) 3-Acetyl-5-methoxy- benzoic acid (DE 2849224)
(IV.1) 1-(4-Methyl-2-pyridyl)-piperazine (US 2979508)
(IV.2) 1-(6-Methoxy-2-pyridyl)-piperazine (J. Med. Chem. 30,7 (1987) 1210-1214)
(IV.3) 1-(3-Ethylphenyl)-piperazine

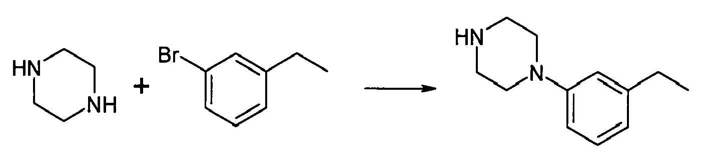

A solution of 1-bromo-3-ethylbenzene (5 g; 27 mmol), piperazine (6.99 g; 81.1 mmol) and potassium *tert*-butylate (9.09 g; 81.05 mmol) in toluene (100 ml) is heated to 80°C under nitrogen atmosphere. Tris-(dibenzylidene acetone)-palladium (74.22 mg; 0.081 mmol) and 2,2'-bis-(diphenylphosphino)-1,1' binaphthyl (BINAP; 0.120 g; 0.189 mmol) are added and the reaction mixture is stirred overnight at 110°C. After removal of the solvent in vacuo and aqueous work-up the crude product is purified by column chromatography (silica; eluent: gradient started with dichloromethane:methanol 7:3 reaching pure dichloromethane) yielding pure 1-(3-ethylphenyl)-piperazine as yellow oil.
Yield: 3.49 g (68 %)
MS: M+1 = 191 Da

### (IV.4) 5-(1-Piperazinyl)-1,3-benzenediol

a) 4-(3,5-Dihydroxy-phenyl)-piperazine-1-carboxylic acid *tert*-butyl ester 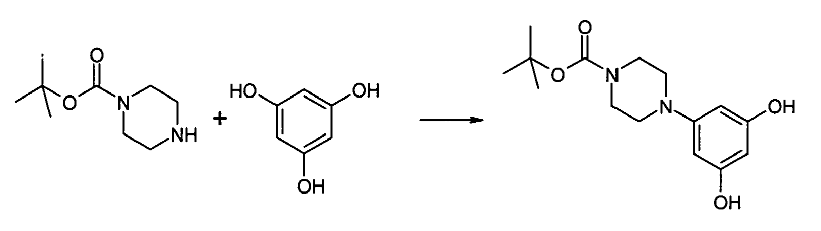 A solution of piperazine-1-carboxylic acid *tert*-butyl ester (6.00 g; 32.2 mmol) and phloroglucine (4.06 g; 32.2 mmol) in toluene (100 ml) is placed in a 250-ml round bottomed flask fitted with a Dean-Stark. The reaction mixture is heated to reflux for 4 h. After cooling to 60°C, the deep red solution is concentrated in vacuo. Addition of dichloromethane to the residue leads to precipitation of the pure product.
   Yield: 4.95 g (52 %)
   HPLC: rt = 3.415 min (Method FEC)
   UV (max.) = 232 nm
   MS: M+1 = 295 Da
b) 5-(1-Piperazinyl)-1,3-benzenediol 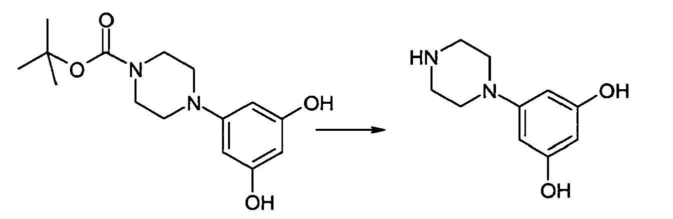 4-(3,5-Dihydroxy-phenyl)-piperazine-1-carboxylic acid *tert*-butyl ester (0.25 g; 0.85 mmol) is dissolved in 10 ml dichloromethane. The solution is cooled down with an ice /sodium chloride mixture and trifluoroacetic acid (0.33 ml; 4.16 mmol) is added while stirring.
   The reaction is allowed to warm up to ambient temperature and stirred for 1 day. The solvent is removed at reduced pressure at 40°C and the remaining crude 5-(1-piperazinyl)-1,3-benzenediol is obtained as oil which needs no further purification.
   HPLC: rt = 0.323 min (Method FEC)
   UV (max.) = 238 nm
   MS: M+1 = 195 Da

### (IV.5) 1-(4-Methoxy-2-pyridinyl)- piperazine

a) 4-(4-Methoxy-2-pyridinyl)-piperazine-1-carboxylic acid *tert*-butyl ester 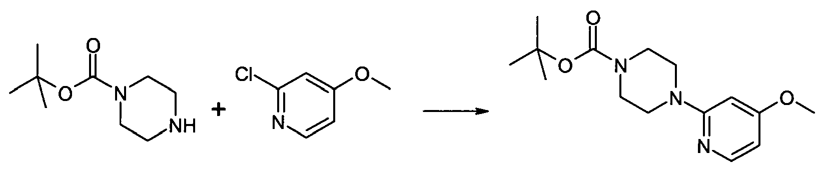 A solution of 2-chloro-4-methoxypyridine (1 g; 6.97 mmol), piperazine-1-carboxylic acid *tert*-butyl ester (3.89 g; 20.9 mmol) and potassium *tert*-butylate (2.34 g; 20.9 mmol) in toluene (60 ml) is heated to 80 °C under nitrogen atmosphere. Tris-(dibenzylidene acetone)-palladium (74.220 mg; 0.081 mmol) and 2,2'-bis-(diphenylphosphino)-1,1' binaphthyl (BINAP; 0.12 g; 0.189 mmol) are added and the reaction mixture is stirred for 3 h at 115°C. After removal of the solvent in vacuo and aqueous work-up the crude product is purified by column chromatography (silica; dichloromethane : methanol 7:3) yielding pure 4-(4-methoxy-2-pyridinyl)-piperazine-1-carboxylic acid *tert*-butyl ester as yellow oil. Yield: 3.49 g (68 %)
   HPLC: rt = 3.056 min (Method FEC)
   UV (max.) = 222 nm
   MS: M+1 = 294 Da
b) 1-(4-Methoxy-2-pyridinyl)-piperazine 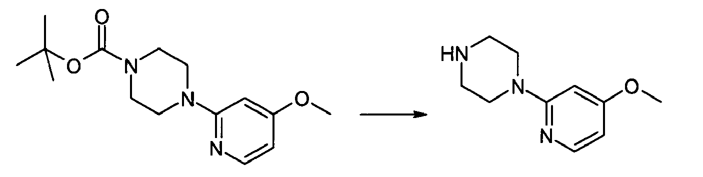 Cleavage of the boc protecting group is done analogously to the procedure as described for intermediate IV.4 and used as starting material for further synthesis.
   Yield: 0.995 g (58 %)
   HPLC: rt = 0.318 min (Method FEC)
   UV (max.) = 230 nm
   MS: M+1 = 194 Da

### (VI.2) 4-Amino-3-diethylaminomethyl-5-chloro-benzoic acid x HCl

a) Ethyl-4-acetylamino-3-methyl-benzoate 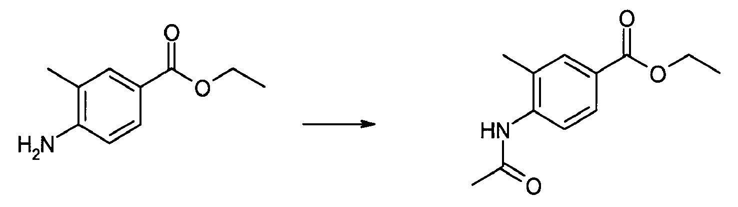 To a suspension of ethyl-4-amino-3-methyl-benzoate (537 g; 3.0 mol; preparation described in Helv. Chim. Acta 62 (1979) 271-303) in 3 litres of toluene is added dropwise acetic anhydride (320 g; 3.14 mol) while stirring at ambient temperature. The reaction mixture is then heated for 2 hours at 85-90°C. The crystals formed upon cooling to ambient temperature are filtered off with suction, dried and recrystallized from ethanol.
   Yield: 590 g (89%)
   Melting point 133-135°C
b) Ethyl-4-acetylamino-3-bromomethyl-benzoate 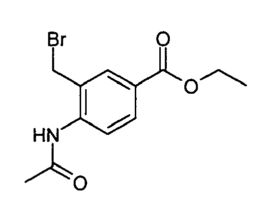 To a solution of ethyl-4-acetylamino-3-methyl-benzoate (22.2.g; 100 mmol) in 1.7 l anhydrous carbon tetrachloride is added NBS (17.5 g; 95 mmol). The solution is irradiated with UV light until TLC indicates complete consumption of NBS (20 min). The crude product crystallizes upon cooling to ambient temperature and is filtered off with suction, dried and further used without purification.
c) Ethyl-4-acetylamino-3-diethylaminomethyl-benzoate 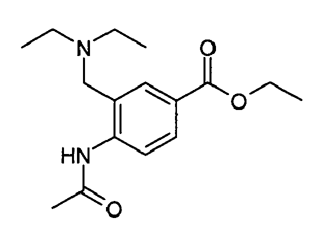 A solution of ethyl-4-acetylamino-3-bromomethyl-benzoate (285 g; 0.95 mol) and diethylamine (148 g; 2.0 mol) in chloroform (2.5 l) is refluxed for 1 hour. After cooling to ambient temperature, the solution is washed twice with water, dried with sodium sulphate and concentrated in vacuo to yield the crude product as oil.
   Yield: 230 g (83%)
   TLC: Rf = 0.55 (chloroform/ethyl acetate 8:2)
d) Ethyl-4-amino-3-diethylaminomethyl-benzoate 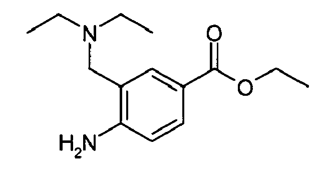 Ethyl-4-acetylamino-3-bromomethyl-benzoate (184 g; 0.63 mol) is refluxed in a mixture of ethanol (600 ml) and hydrochloride acid (6 N; 600 ml) for 1 hour. The solution is concentrated to a volume of 400 ml, poured on ice, made alkaline by addition of sodium hydroxide, and extracted with diethyl ether. The organic layer is dried with sodium sulphate and concentrated in vacuo to yield oil.
   Yield: 98 g (62%)
   m.p. of the hydrochloride after recrystallization from ethanol: 194-200°C
e) Ethyl-4-amino-3-diethylaminomethyl-5-chloro-benzoate x HCl 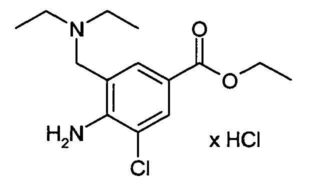 A solution of ethyl-4-amino-3-diethylaminomethyl-benzoate (98 g; 0.392 mol) in acetic acid (95%; 300 ml) is cooled to 7°C. A freshly prepared solution of chlorine (27.8 g; 0.392 mol) in acetic acid (95%; 400 ml) is added rapidly while shaking. After 30 seconds the reaction mixture is poured on ice-water (1.5 l), and made alkaline by the addition of ammonia. The product is extracted with chloroform and purified by column chromatography (silica, chloroform: ethyl acetate 9:1) to yield an oil which is converted to the hydrochloride by the addition of HCl in ethanol.
   Yield: 60.6 g (48%)
   Melting point (after repeated recrystallization from ethanol): 153-156°C
f) 4-Amino-3-diethylaminomethyl-5-chloro-benzoic acid x HCl 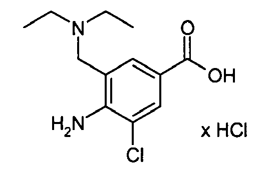 Ethyl-4-amino-3-diethylaminomethyl-5-chloro-benzoate x HCl (50.5 g; 0.157 mol) is refluxed in hydrochloric acid (6 mol/1; 700 ml) for 1 hour. The solution is concentrated to a volume of 200 ml from which the product crystallizes after cooling to ambient temperature.
   Yield: 20 g (44%)
   Melting point: 230-234°C

### (VI.3) 4-Amino-3-cyano-5-fluoro-benzoic acid

a) Ethyl-4-amino-3-cyano-5-fluoro-benzoate 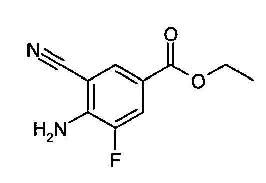 Ethyl-4-amino-3-cyano-5-fluoro-benzoate is prepared from ethyl-4-amino-3-fluorobenzoate analogously to the synthesis of 4-acetyl-2-cyano-6-fluoro-anilin as described in Drug Research 34, 11a (1984); 1612-1624.
b) 4-Amino-3-cyano-5-fluoro-benzoic acid 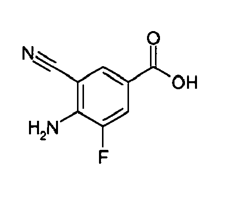 Ethyl-4-amino-3-cyano-5-fluoro-benzoate (10.4 g; 0.05 mol) is added to a solution of sodium hydroxide (2.0 g; 55 mmol) in 20 ml water and 200 ml methanol. The mixture is stirred for 24 hours at ambient temperature, then water (300 ml) is added, and unreacted ester is removed by extraction with ethyl acetate. The aqueous layer is acidified with KHSO₄ and extracted with ethyl acetate. The organic layer is dried over sodium sulphate, filtered through activated charcoal, and concentrated to dryness. The crude product is recrystallized from 2-propanol.
   Yield: 3.6 g (40%)
   Melting point: 281-283°C

### (VII.1) 1-(2,2-Difluoro-benzo[1,3]dioxol-4-yl)-piperazine

a) 4-(2,2-Difluoro-benzo[1,3]dioxol-4-yl)-piperazine-1-carboxylic acid *tert-*butyl ester 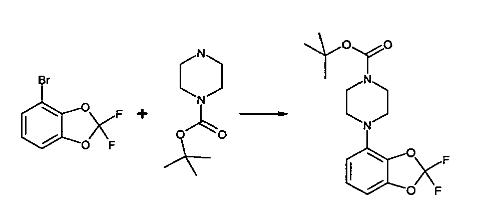 Piperazine-1-carboxylic acid *tert-*butyl ester (50.6 mmol; 9.43 g), potassium-*tert*-butylate (50.6 mmol; 5.68 g) and 4-bromo-2,2-difluoro-1,3-benzdioxole (8.4 mmol; 2 g) are suspended in 80 ml toluene. The stirred mixture is heated to 80°C prior to the addition of BINAP (0.06 mmol; 39 mg) and tris-(dibenzylidenaceton)-palladium (0) (0.022 mmol; 19.8 mg). After additional 5 hours at 115 °C, the reaction is cool down and the solvent is removed in vacuo at 60°C. The residue is mixed with water and extracted three times with ethyl acetate.
   The combined organic layers are dried over magnesium sulfate and the solvent is removed at 40°C in vacuo. The obtained brownish oil is purified by reversed phase chromatography using an acetonitrile / water gradient. After removal of the solvent, the product is obtained as yellow oil.
   Yield: 1.29 g (44.6%)
   HPLC: rt = 5.383 min (Method FEC)
   UV (max.) = 250 nm
   MS: M+1= 343 Da
b) 1-(2,2-Difluoro-benzo[1,3]dioxol-4-yl)-piperazine 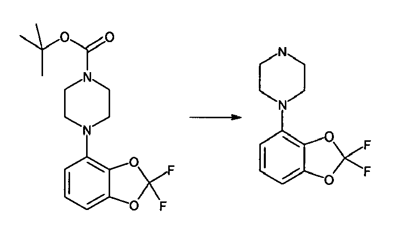 3.77 mmol (1.29 g) 4-(2,2-Difluoro-benzo[1,3]dioxol-4-yl)-piperazine-1-carboxylic acid *tert*-butyl ester are dissolved in 30 ml dichloromethane. The solution is cooled down with an ice / sodium chloride mixture and 18.8 mmol (1.4 ml) trifluoroacetic acid are added while stirring.
   The reaction is allowed to warm up to ambient temperature and stirred for 3 days. The solvent is removed at reduced pressure at 40°C and the remaining crude product is dissolved in water. Saturated sodium carbonate is added until a pH value above 9 is achieved. The solution is extracted three times with dichloromethane. The combined organic layers are dried over magnesium sulfate and the solvent is removed under reduced pressure. 1-(2,2-Difluoro-benzo[1,3]dioxol-4-yl)-piperazine is obtained as oil and needs no further purification.
   Yield: 494 mg (54.1 %)
   HPLC: rt = 0.363 min (Method FEC)
   UV (max.) = 242 nm
   MS:M+1=243Da
   Following intermediates are prepared analogously to the procedure as described for intermediate VII. 1.

### (VII.2) Dimethyl-(3-piperazin-1-yl-phenyl)-amine

a) 4-[3-(2-Hydroxy-ethyl)-phenyl]-piperazine-1-carboxylic acid *tert*-butyl ester 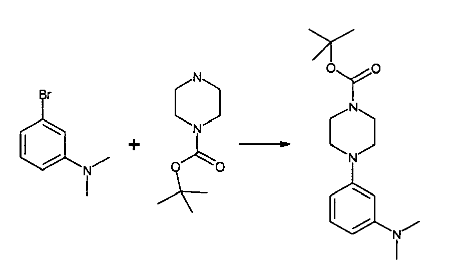 Yield: 95 mg (7.1 %)
   HPLC: rt = 3.987 min (Method FEC)
   UV (max.) =258 nm
   MS: M+1 = 306 Da
b) Dimethyl-(3-piperazin-1-yl-phenyl)-amine 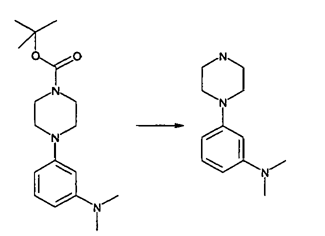 The cleavage of the boc protecting group is done analogously to the procedure as described for intermediate VII. 1 and the crude product is used as starting material for further syntheses.
   HPLC: rt = 0.287 min (Method FEC)
   UV (max.) = 250 nm
   MS: M+1 = 206 Da

### (VII.3) 2-(3-Piperazin-1-yl-phenyl)-ethanol

a) 4-[3-(2-Hydroxy-ethyl)-phenyl]-piperazine-1-carboxylic acid *tert*-butyl ester 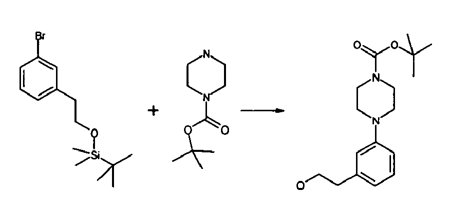 Piperazine-1-carboxylic acid *tert*-butyl ester (29.9 mmol; 5.564 g) is dissolved in 80 ml toluene. To the solution potassium-*tert*-butylate (29.9 mmol; 3.352 g) and [2-(3-bromophenyl)-ethoxy]-*tert*-butyl-dimethyl-silane (10 mmol; 3.14 g) are added. The solution is stirred at 80°C and BINAP (0.074 mmol; 46 mg) followed by tris-(dibenzylidenaceton)-palladium (0) (0.026 mmol; 23.8 mg) are added. The reaction mixture is heated at 115°C for 3 hours. After cooling, the solvent is removed under reduced pressure at 60°C. The residue is mixed with water and extracted 3 times with ethyl acetate. The combined organic layers are dried over magnesium sulfate and the solvent is removed at 40°C in vacuo.
   The obtained brownish oil is treated with 70 ml of an acetic acid / water / THF mixture (3:1:1) for 15 hours at room temperature. After removal of the solvent under reduced pressure an oil is obtained which is purified by reversed phase chromatography using an acetonitrile / water gradient. After evaporation of the solvent the pure compound is obtained.
   Yield: 1.78 g (58.3 %)
   HPLC: rt = 4.504 min (Method FEC)
   UV (max.) = 250 nm
   MS: M+1 = 307 Da
b) 2-(3-Piperazin-1-yl-phenyl)-ethanol 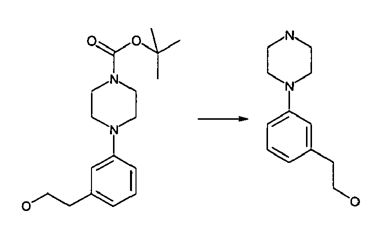 Cleavage of the boc protecting group is done analogously to the procedure as described for intermediate VII.1.
   Yield: 150 mg (89.1%)
   HPLC: rt = 0.646 min (Method FEC)
   UV (max.) = 242 nm
   MS: M+1 = 207 Da

### (VII.4) 1-(3,5-Dimethoxy-phenyl)-[1,4]diazepane

a) 4-(3,5-Dihydroxy-phenyl)-[1,4]diazepane-1-carboxylic acid *tert*-butyl ester 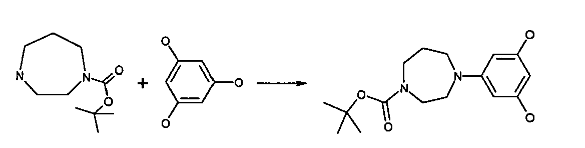 Homopiperazin-1-carboxylic acid *tert*-butyl ester (10.74 mmol; 2.15 g) is dissolved in 50 ml toluene. To the solution phloroglucin (10.74 mmol; 1.354 g) is added and the reaction mixture is stirred under reflux. After completion of the reaction the red solvent is filtered off from the precipitated product.
   Yield: 2.635 g (79.6 %)
   HPLC: rt = 3.943 min (Method FEC)
   UV (max.) = 226 nm
   MS: M+1 = 309 Da
b) 4-(3,5-Dimethoxy-phenyl)-[1,4]diazepane-1-carboxylic acid *tert*-butyl ester 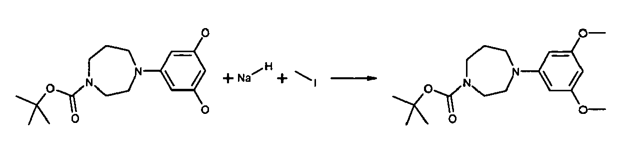 1.62 mmol (500 mg) of -(3,5-dihydroxy-phenyl)-[1,4]diazepane-1-carboxylic acid *tert-*butyl ester are dissolved in 10 ml N,N-dimethylformamide. To the solution 3.56 mmol (0.142 g) sodium hydride are added and stirred for 5 min. 3.56 mmol (0.221 ml) Methyl-iodide are added dropwise and stirred for 20 h at room temperature.
   The reaction is quenched with aqueous ammonia and the solvent is removed under reduced pressure at 60°C. The crude product is chromatographed over a reversed phase column using an acetonitrile water gradient. Fractions containing the desired product are pooled and the solvent is removed in vacuo.
   Yield: 125 mg (22.9%)
   HPLC: rt = 4,845 min (Method FEC)
   UV (max.) = 222 nm
   MS: M+1 = 337 Da
d) 1-(3,5-Dimethoxy-phenyl)-[1,4]diazepane 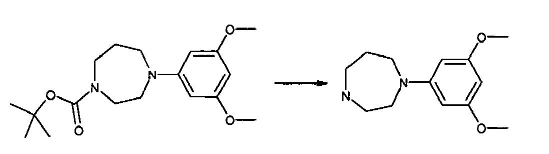 Cleavage of the boc protecting group is done analogously to the procedure as described for intermediate VII. 1 and the product is used as starting material for further syntheses.
   HPLC: rt = 0.336 min (Method FEC)
   UV (max.) = 218 nm
   MS: M+1 = 237 Da

### (VII.5) 2-Methoxy-6-piperazin-1-yl-benzonitrile

a) 4-(2-Cyano-3-methoxy-phenyl)-piperazine-1-carboxylic acid *tert*-butyl ester 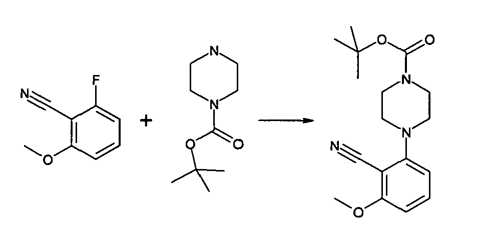 2-Fluoro-6-methoxybenzonitrile (9.93 mmol; 1.5 g) is dissolved in 24 ml 1.4-dioxane. To the solution piperazine-1-carboxylic acid *tert*-butyl ester (9.93 mmol; 1.85 g) and 3.4 ml Hünigs base are added.
   The solution is filled in reaction tubes, sealed and irradiated in a microwave oven (180°C) for 30 minutes. The yellowish reaction solution is concentrated under reduced pressure and purified by reversed phase chromatography using an acetonitrile / water gradient. Fractions containing the desired product are pooled and the solvent is removed in vacuo at 40°C in order to obtain the product as oil.
   Yield: 102 mg (3.2%)
   HPLC: rt = 5.124 min (Method FEC)
   UV (max.) = 226 nm
   MS: 262 Da (Carbaminic acid fragment)
b) 2-Methoxy-6-piperazin-1-yl-benzonitrile 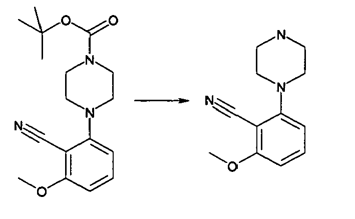 Cleavage of the boc protecting group is done analogously to the procedure as described for intermediate VII. 1 and the product is used as starting material for further syntheses.
   HPLC: rt = 1.827 min (Method FEC)
   UV (max.) = 222 nm
   MS: M+1 = 218 Da

### (VII.6) 1-[3-(2,2,2-Trifluoro-ethoxy)-phenyl]-piperazine

a) 4-[3-(2,2,2-Trifluoro-ethoxy)-phenyl]-piperazine-1-carboxylic acid *tert*-butyl ester 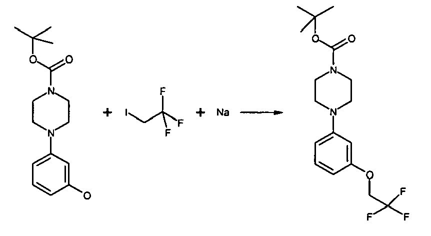 3.31 mmol (920 mg) of 4-(3-hydroxy-phenyl)-piperazine-1-carboxylic acid *tert*-butyl ester are dissolved in 20 ml N,N-dimethylformamide and sodium hydride (3.31 mmol; 123 mg) is added under stirring. 2-Iodo-trifluoroethane (3.31 mmol; 326 *µ*l) and potassium carbonate (6.61 mmol; 914 mg) are added after 5 minutes and aliquots of the reaction mixture are filled in sealed tubes. After irradiation in a microwave oven (150°C) for 20 minutes 2 equivalents of 2-iodo-trifluoroethane are added and the irradiation step is repeated.
   The combined reaction solution is quenched with aqueous ammonia for 30 minutes and the solvent is removed under reduced pressure. Water is added to the remaining brown oil and the mixture is extracted 3 times with acetylethylester. The combined organic layers are dried over sodium sulfate and the solvent is removed in vacuo. The final purification of the obtained oil is achieved by reversed phase chromatography using an acetonitrile / water gradient. Fractions containing the desired product are concentrated in vacuo in order to obtain the pure product.
   Yield: 362 mg (30.4 %)
   HPLC: rt = 5.172 min (Method FEC)
   UV (max.) = 252 nm
   MS: M+1 = 361 Da
b) 1-[3-(2,2,2-Trifluoro-ethoxy)-phenyl]-piperazine 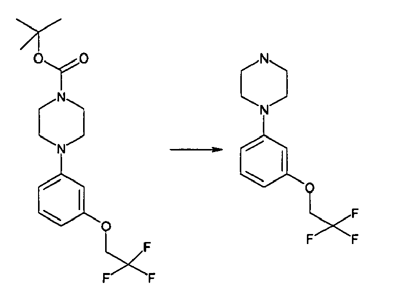 Cleavage of the boc protecting group is done analogously to the procedure as described for intermediate VII. 1
   Yield: 260 mg (99.5%)
   HPLC: rt = 3,283 min (Method FEC)
   UV (max.) = 246 nm
   MS: M+1 = 261 Da

### (VII.7) 3-Methoxy-5-piperazin-1-yl-phenol

a) 4-(3-Hydroxy-5-methoxy-phenyl)-piperazine-1-carboxylic acid *tert*-butyl ester 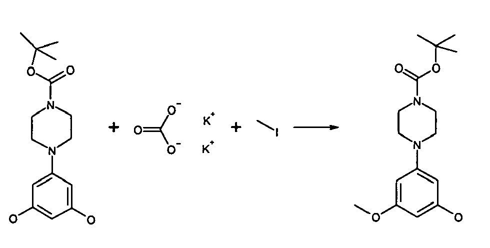 The compound is prepared analogously as described for intermediate VII.4 using potassium carbonate as base.
   HPLC: rt = 4.284 min (Method FEC)
   UV (max.) = 218 nm
   MS: M+1 = 308 Da
b) 3-Methoxy-5-piperazin-1-yl-phenol 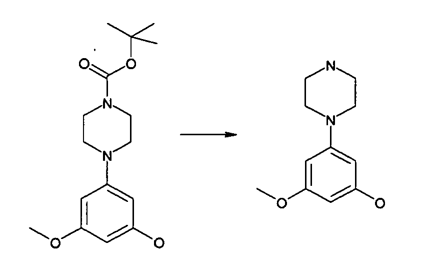 Cleavage of the boc protecting group is done analogously to the procedure as described for intermediate VII. 1 and used as starting material.

### (VII.8) 1-(3,5-Bis-difluoromethoxy-phenyl)-piperazine and

### (VII.9) 3-Difluoromethoxy-5-piperazin-1-yl-phenol

a) 4-(3-Difluoromethoxy-5-hydroxy-phenyl)-piperazine-1-carboxylic acid *tert*-butyl ester and 4-(3,5-Bis-difluoromethoxy-phenyl)-piperazine-1-carboxylic acid *tert*-butyl ester 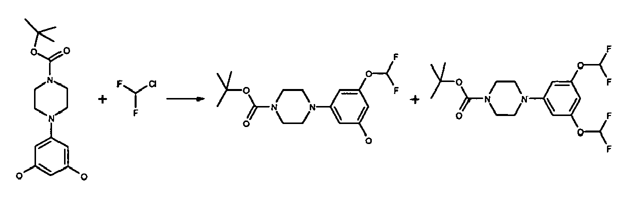 4-(3,5-Dihydroxy-phenyl)-piperazine-1-carboxylic acid *tert*-butyl ester (5.1 mmol; 1.5 g) is dissolved in a mixture of 20 ml isopropanol and 20 ml aqueous sodium hydroxide (20%) in an autoclave. A pressure of 50 psi is build up with chloro-difluoro-methane and maintained for 3 hours at 60°C. After completion of the reaction the solvent is removed in vacuo and the residue is a mixture of the two desired compounds. The two compounds are separated from each other by reversed phase chromatography using an acetonitrile / water gradient. Fractions containing the respective pure product are pooled and the solvent is removed in vacuo.
   Yield: 303 mg (17.3%) (VII.8)
   HPLC: rt = 4.819 min (Method FEC)
   UV (max.) = 214 nm
   MS: M+1 = 345 Da
   Yield: 438 mg (21.8%) (VII.9)
   HPLC: rt = 5.923 min (Method FEC)
   UV (max.) = 250 nm
   MS: M-BOC = 295 Da

1-(3,5-Bis-difluoromethoxy-phenyl)-piperazine and
3-difluoromethoxy-5-piperazin-1-yl-phenol 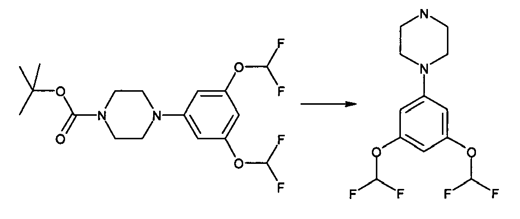 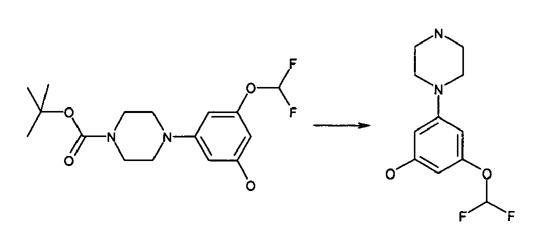 Cleavage of the boc protecting group is done analogously to the procedure as described for intermediate VII. 1 and the obtained products are used as starting material for further syntheses.
HPLC: rt = 4.164 min (Method FEC)
UV (max.) = 250 nm
MS: M+1 = 295 Da
HPLC: rt =1.320 min (Method FEC)
UV (max.) = 246 nm
MS: M+1 = 245 Da

### (VII.10) 1-(3-Allyloxy-phenyl)-piperazine

a) 4-(3-Allyloxy-phenyl)-piperazine-1-carboxylic acid *tert-*butyl ester 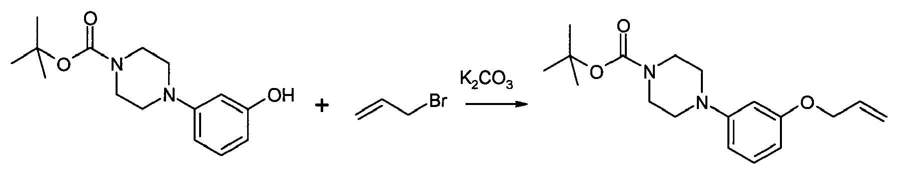 4-(3-Hydroxy-phenyl)-piperazine-1-carboxylic acid *tert*-butyl ester (3.6 mmol; 1 g) is dissolved in 20 ml DMF and potassium carbonate (7.2 mmol; 0.99 g) is added under stirring. Allyl bromide (3.6 mmol; 0.3 ml) is added and the reaction mixture is stirred at RT for 2 days. After aqueous work-up the combined organic phases are dried over sodium sulphate and filtered. Removal of the solvent in vacuo yields pure product as a light-brown oil
   Yield: 1.12 g (98%)
   HPLC: rt = 4.603 min (Method A)
   UV (max) = 250 nm
   MS: [M+H]⁺ = 319
b) 1-(3-Allyloxy-phenyl)-piperazine 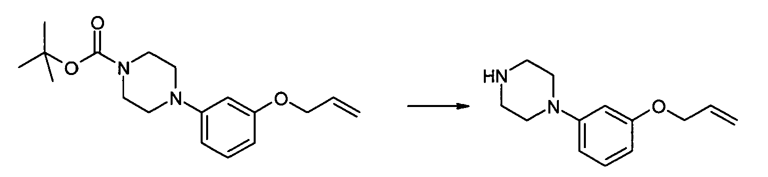 4-(3-Allyloxy-phenyl)-piperazine-1-carboxylic acid *tert*-butyl ester (3.5 mmol; 1.1 g) is dissolved in dichloromethane (40 ml) and cooled to 0°C. TFA is added (17.3 mmol; 1.33 ml) and the reaction mixture is stirred overnight at RT. In order to complete the reaction, an additional portion of TFA (0.5 ml) is added. After 5h at RT, the solvent is evaporated and the pure product is obtained.
   Yield: 1.54 g (95%)
   HPLC: rt = 2.883 min (Method A)
   UV (max) = 245 nm
   MS: [M+H]⁺ = 219

### (VII.11) 1-(3-Allyloxy-5-hydroxy-phenyl)-piperazine and

### (VII.12) 1-(3,5-Diallyloxy-phenyl)-piperazine

a) 1-(3-Allyloxy-5-hydroxy-phenyl)-piperazine-1-carboxylic acid *tert*-butyl ester and 1-(3,5-Bis-allyloxy-phenyl)-piperazine-1-carboxylic acid *tert*-butyl ester 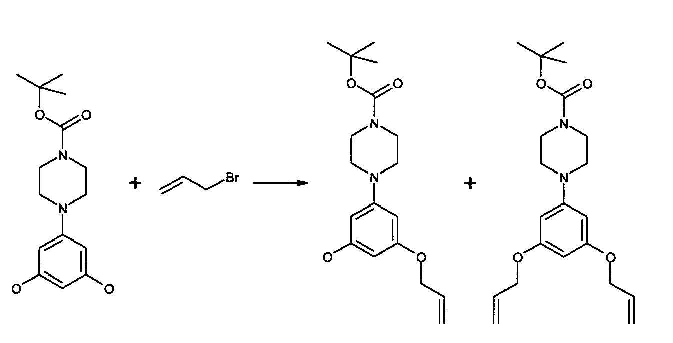 4-(3,5-Dihydroxy-phenyl)-piperazine-1-carboxylic acid *tert-*butyl ester (9.8 mmol; 2.9 g) is dissolved in 60 ml DMF and potassium carbonate (9.8 mmol; 1.3 g) is added under stirring. Allyl bromide (9.8 mmol; 0.83 ml) is added and the reaction mixture is stirred overnight at RT. An additional equivalent potassium carbonate (9.8 mmol; 1.3 g) and allyl bromide (9.8 mmol; 0.83 ml) are added and the mixture is stirred for further 5 days at RT. After addition of aqueous ammonia (20 ml) the mixture is stirred for 10 min until the solvent is evaporated under reduced pressure. The residue - HPLC/MS-analysis indicated a 1:1 mixture of mono- and bis-allylated product ― is purified by reversed phase chromatography (RP-silica, eluent: gradient started with acetonitrile/water 95:5, reaching 5:95 within 30 min).
   Yield: 475 mg (14 %)
   HPLC: rt = 3.999 min (Method A)
   UV (max) = 218 nm
   MS: [M+H]⁺ = 335
   Yield: 660 mg (18 %)
   HPLC: rt = 4.850 min (Method A)
   UV (max) = 226 nm
   MS: [M+H]⁺ = 375
b) 1-(3-Allyloxy-5-hydroxy-phenyl)-piperazine 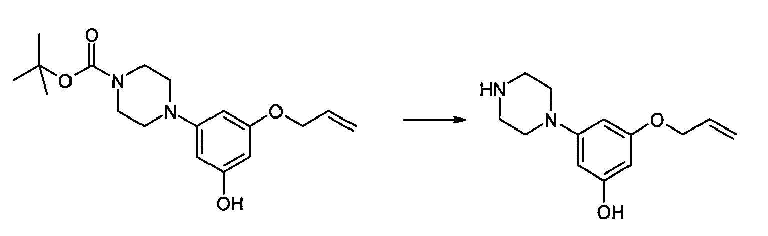 1-(3-Allyloxy-5-hydroxy-phenyl)-piperazine-1-carboxylic acid *tert*-butyl ester (1.35 mmol; 450 mg) is dissolved in dioxane (2 ml), HCl (4 M in dioxane; 5 ml) is added and the reaction mixture is stirred at RT for 3h. Concentration in vacuo afforded pure product. Yield: 400 mg (98 %)
   HPLC: rt = 1.607 min (Method A)
   UV (max) = 214 nm
   MS: [M+H]⁺ = 235
b) 1-(3,5-Bis-allyloxy-phenyl)-piperazine 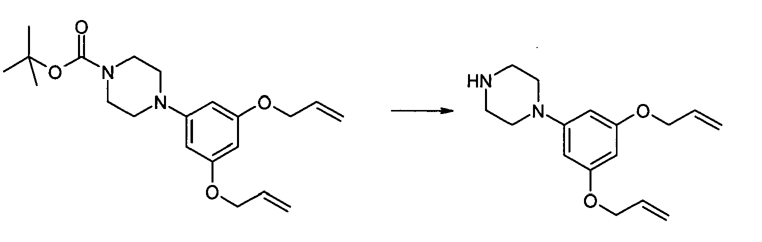 Cleavage of the boc protecting group is done analogously to the procedure as described for intermediate VII.11 and the obtained product is used as starting material for further syntheses.
   Yield: 400 mg (98 %)
   HPLC: rt = 1.607 min (Method A)
   UV (max) = 220 nm
   MS: [M+H]⁺ = 275

### (VIII.1) 1-(3,5-Dimethoxyphenyl)-4-(2-iodobenzoyl)-piperazine

The compound is prepared analogously to the procedure as described for example 1.1 starting from compounds 1.23 and II.9.

### Examples:

### Example 1.1

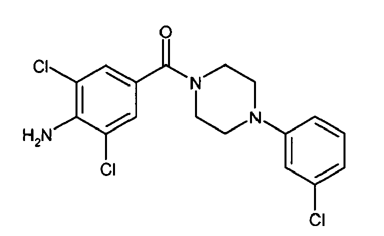

4-Amino-3,5-dichloro-benzoic acid (compound 1.1; 1.03 g; 5.0 mmol), 1-(3-chlorophenyl)-piperazine (compound II.1; 0.983 g; 5.0 mmol, triethylamine (0.759 ml; 7.5 mmol) are dissolved in DMF (20 ml). After addition of TBTU (1.605 g; 5.0 mmol) is the solution stirred overnight at 50 °C. After addition of water, extraction with ethyl acetate and evaporation of the organic layer, the crude product is purified by column chromatography (silica, eluent: DCM/Methanol 50:2).
Yield: 0.9 g (47 %; colourless solid)
¹H-NMR (DMSO) d = 3.20 (br s, 4H), 3.60 (br s, 4H), 5.89 (br s, 2H), 6.80 (d, 1H), 6.89 (d, 1H), 6.94 (s, 1H), 7.21 (t, 1H), 7.35 (s, 2H).
Rf value TLC: 0.25 (Silica; DCM)
Mass Spectrum: m/z = 384/386/388 [M+H]⁺ (C₁₇H₁₆Cl₃N₃O)

Following compounds are prepared analogously to the procedure described for example 1.1 using the starting materials given in the table.

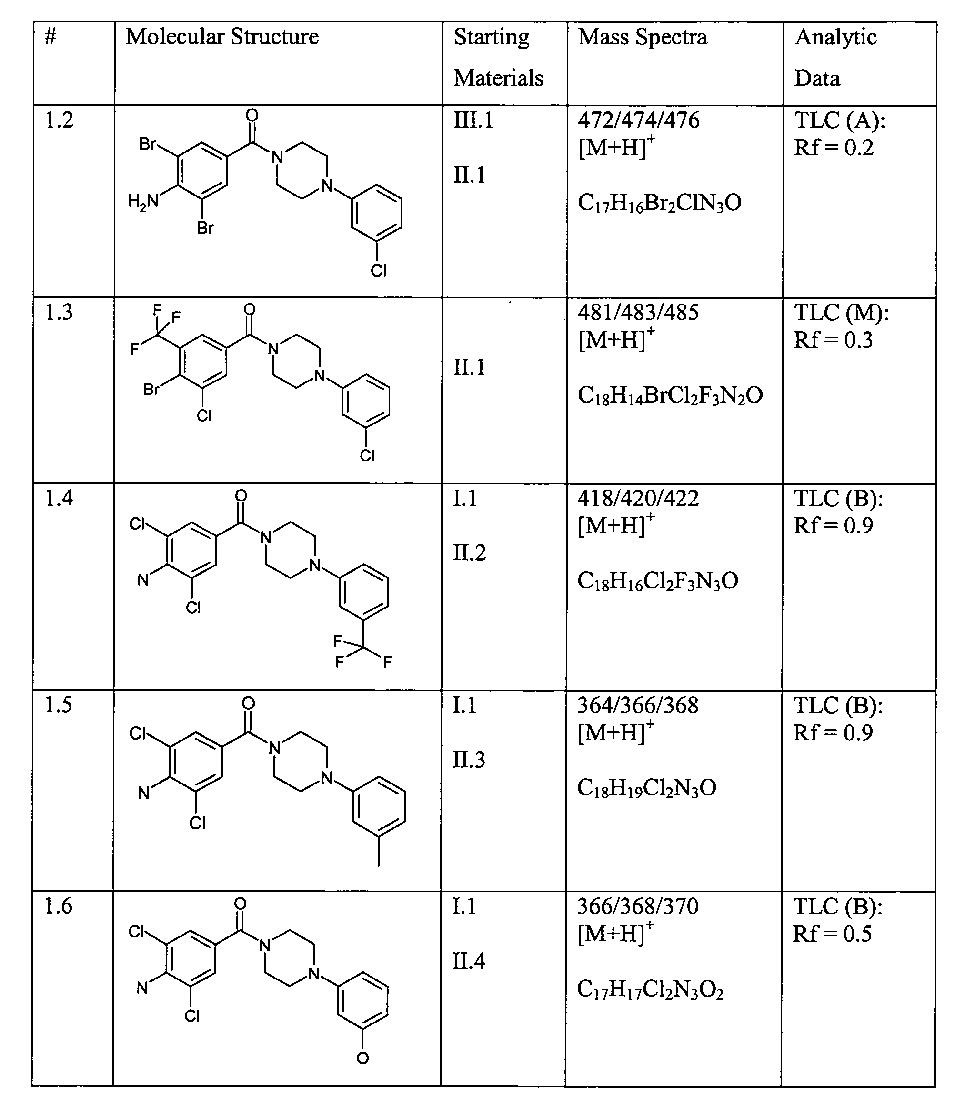

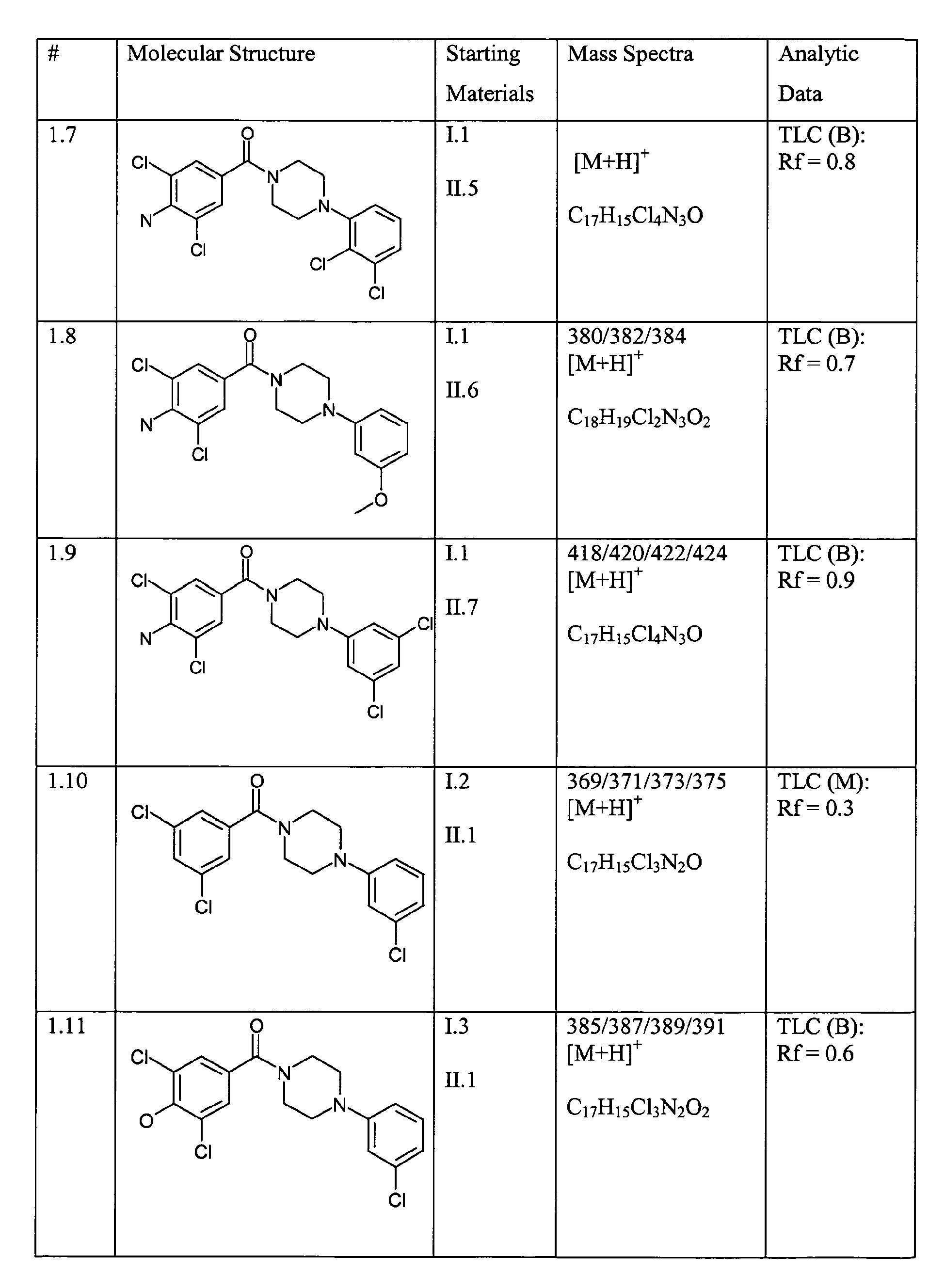

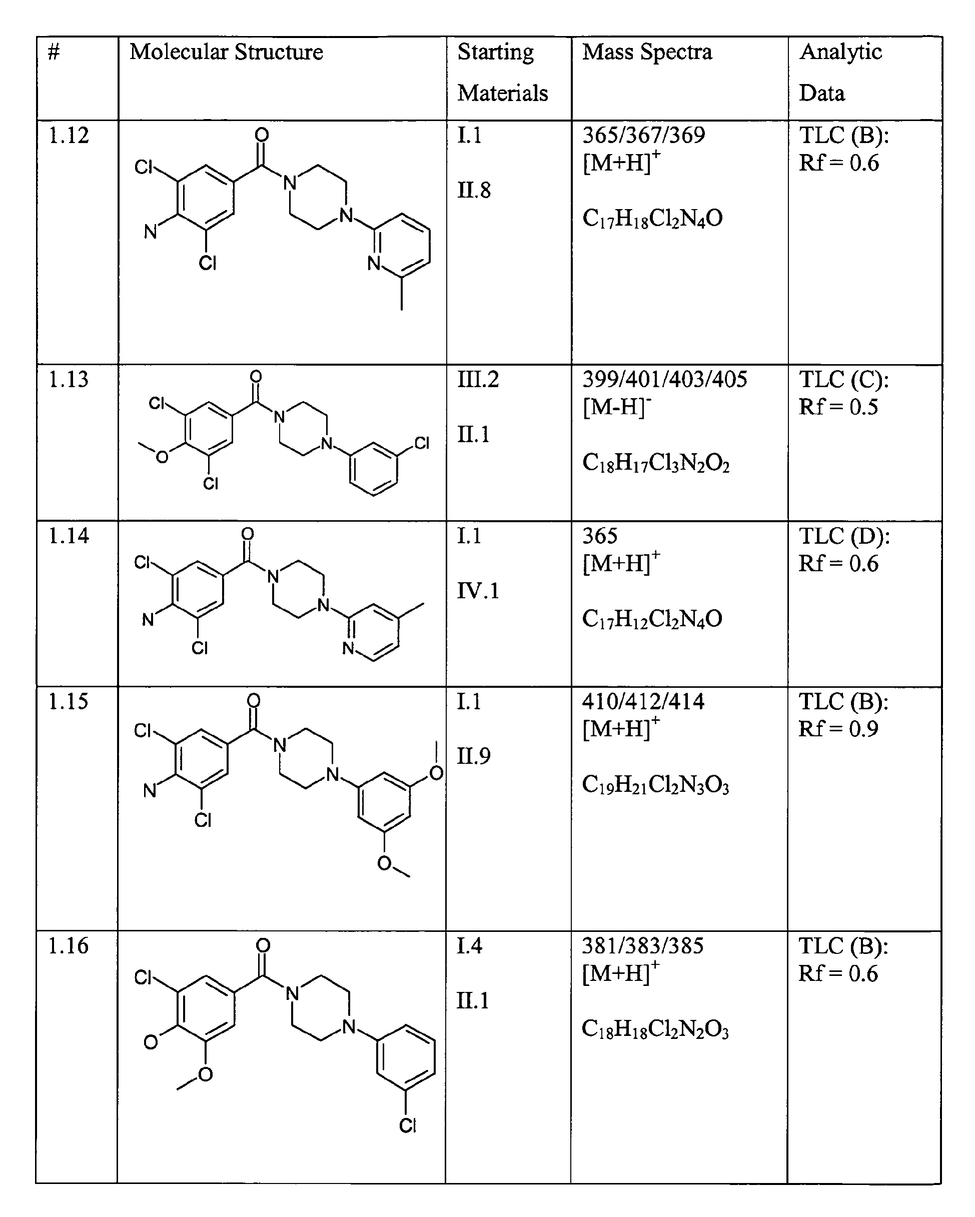

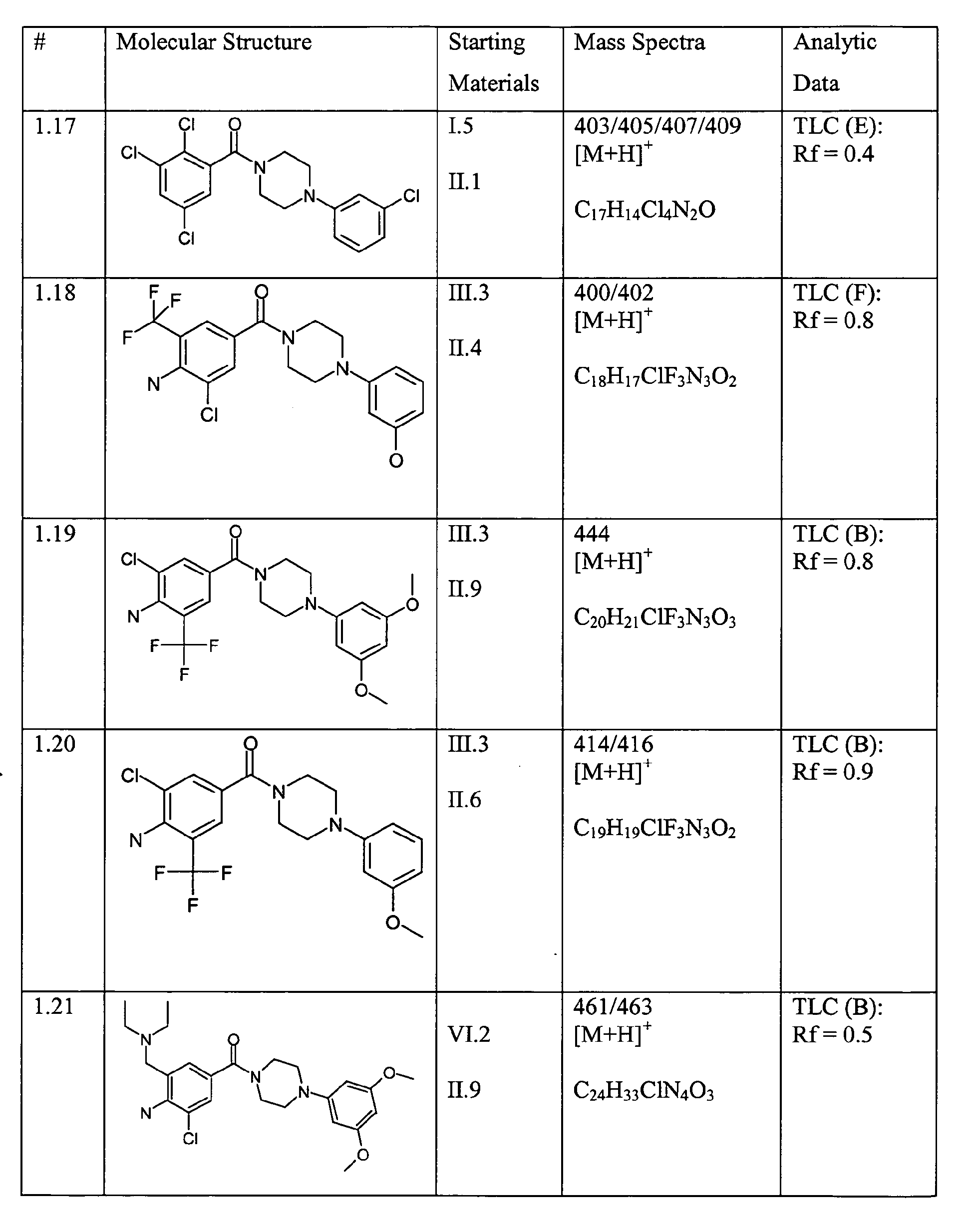

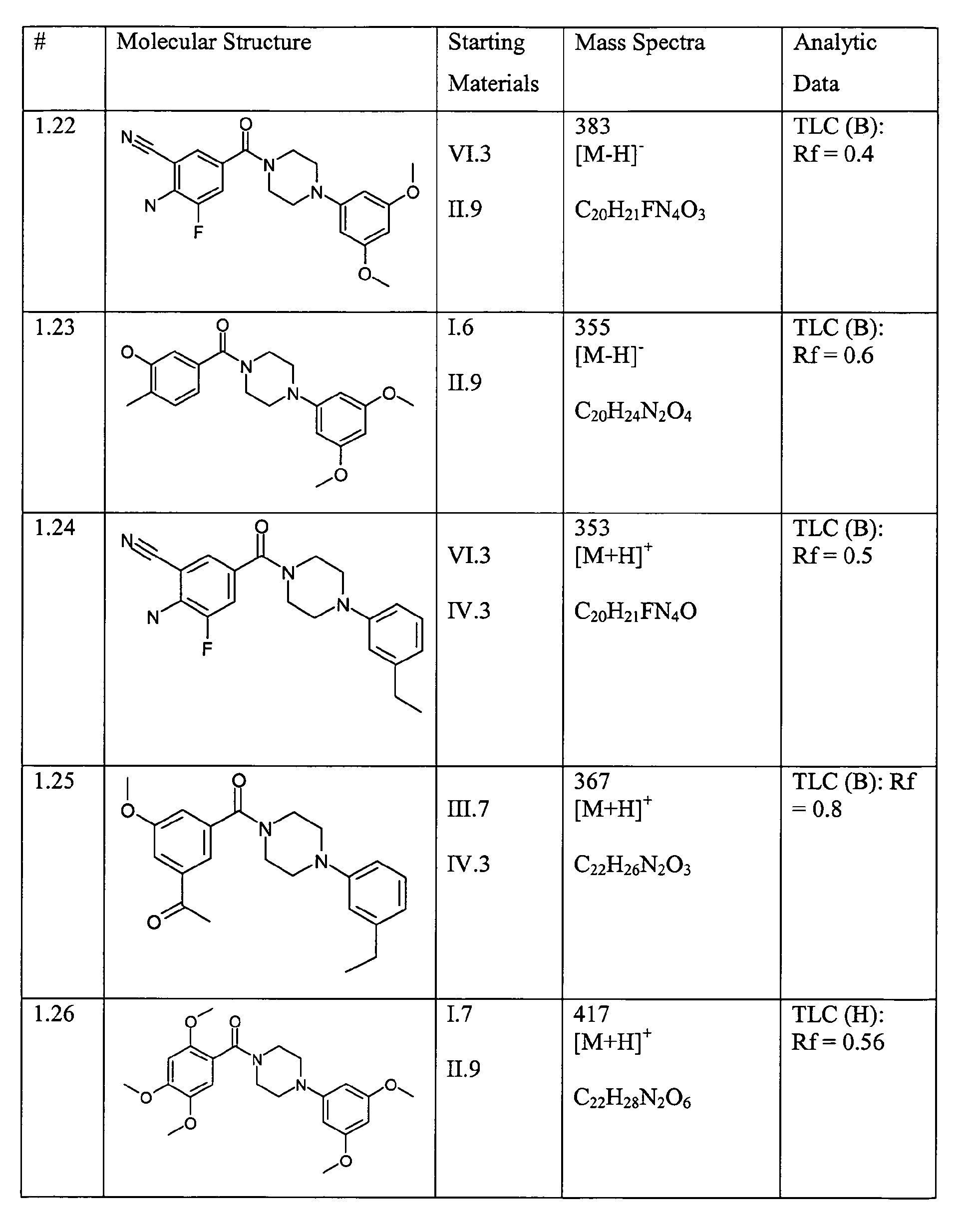

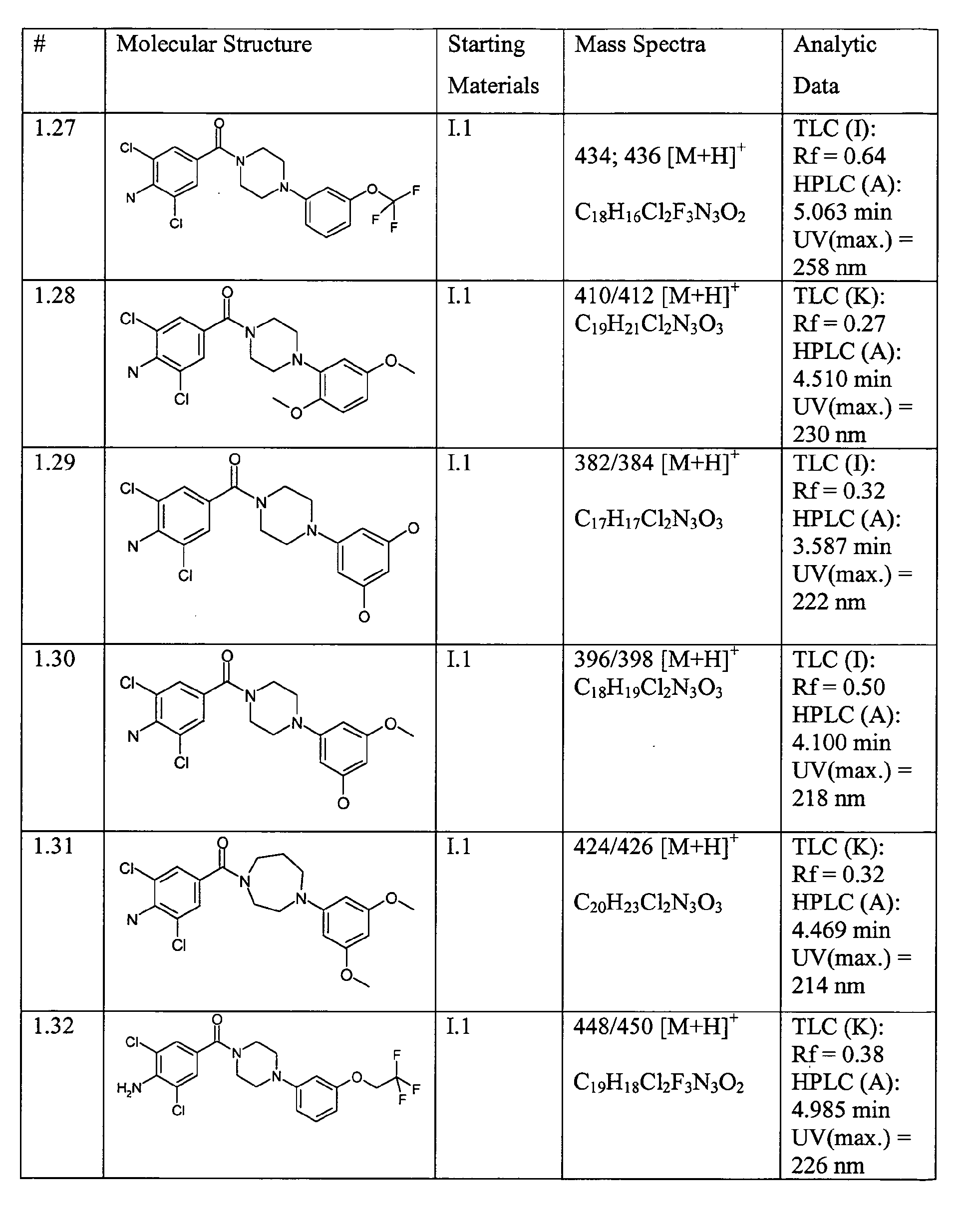

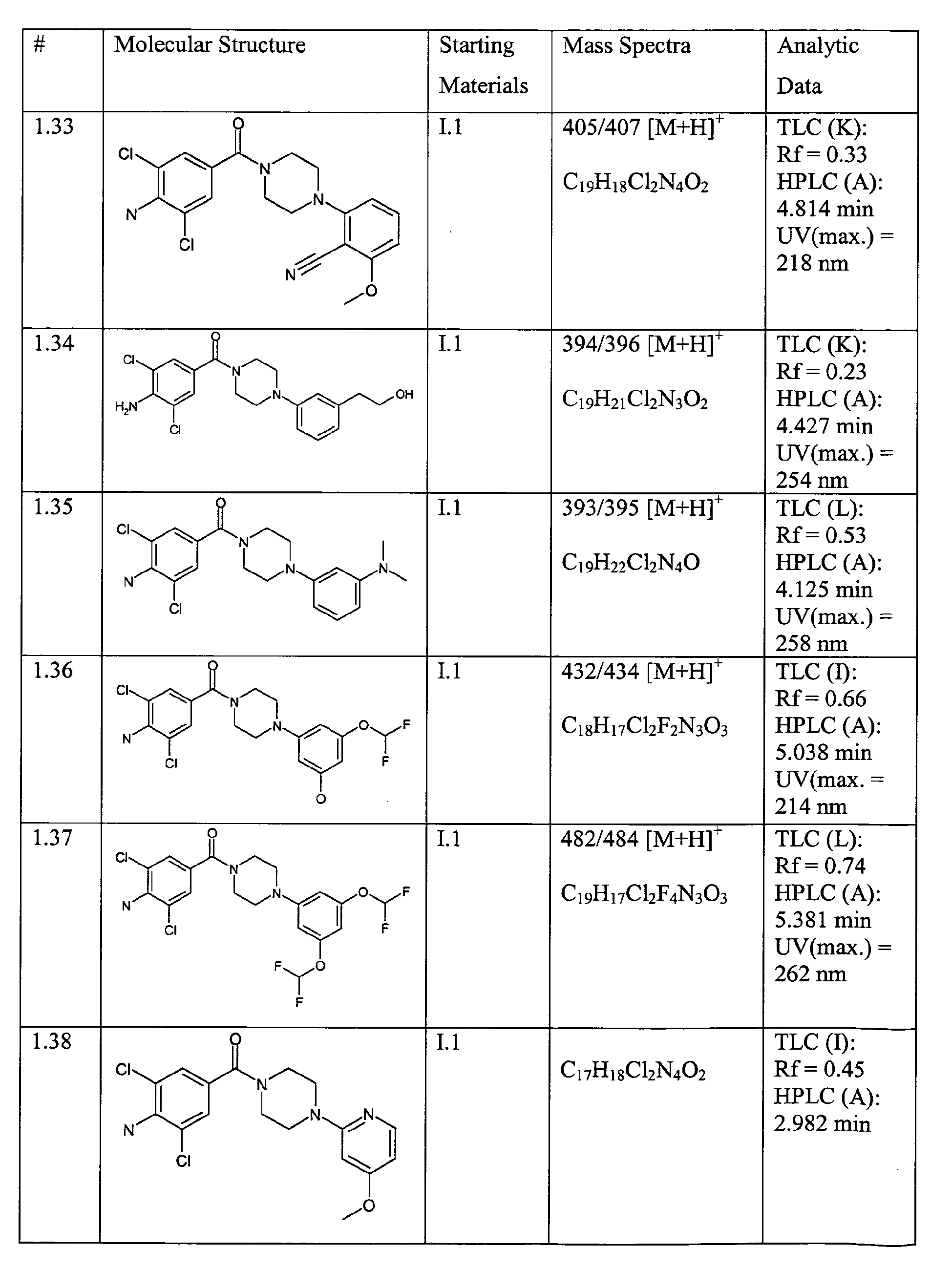

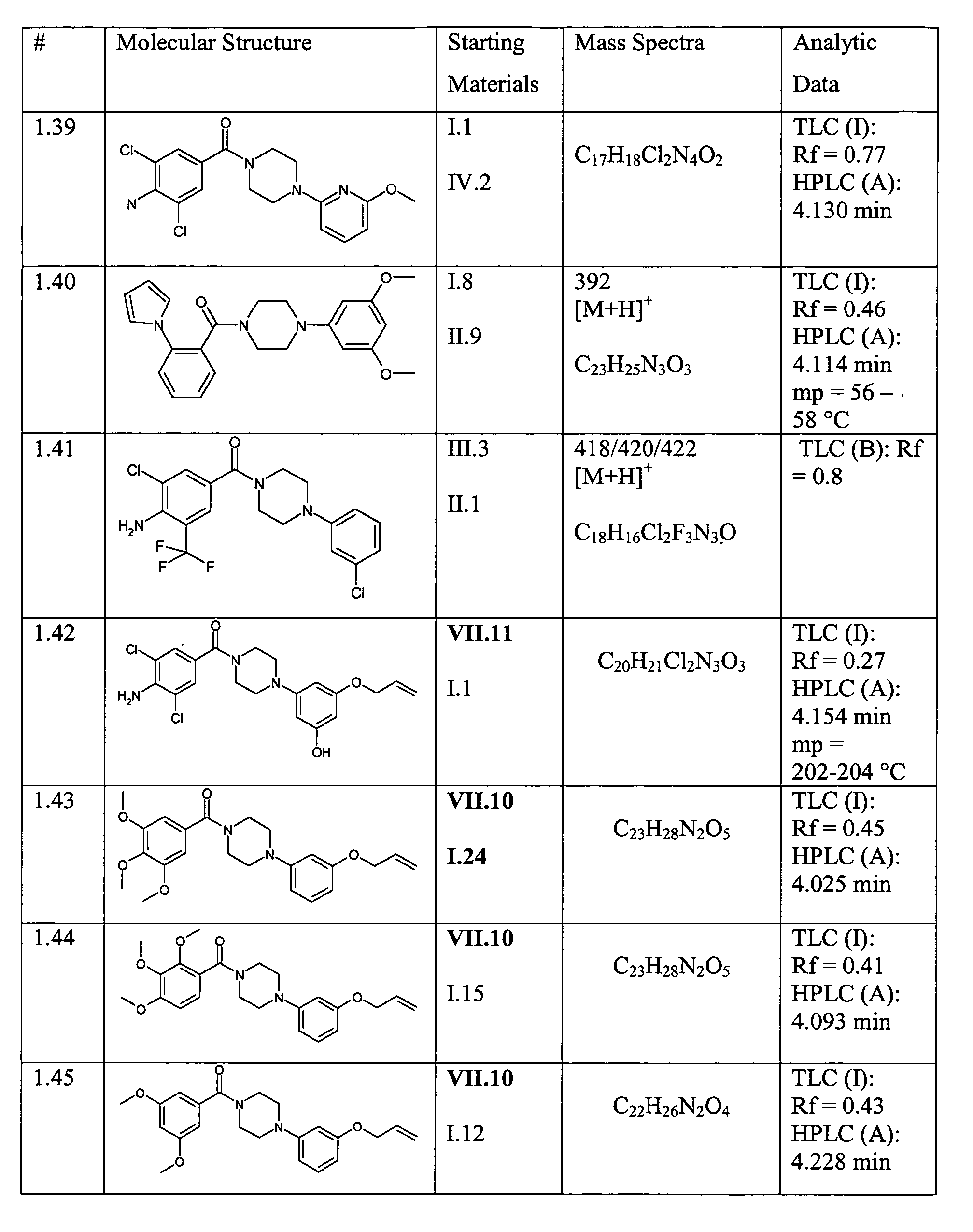

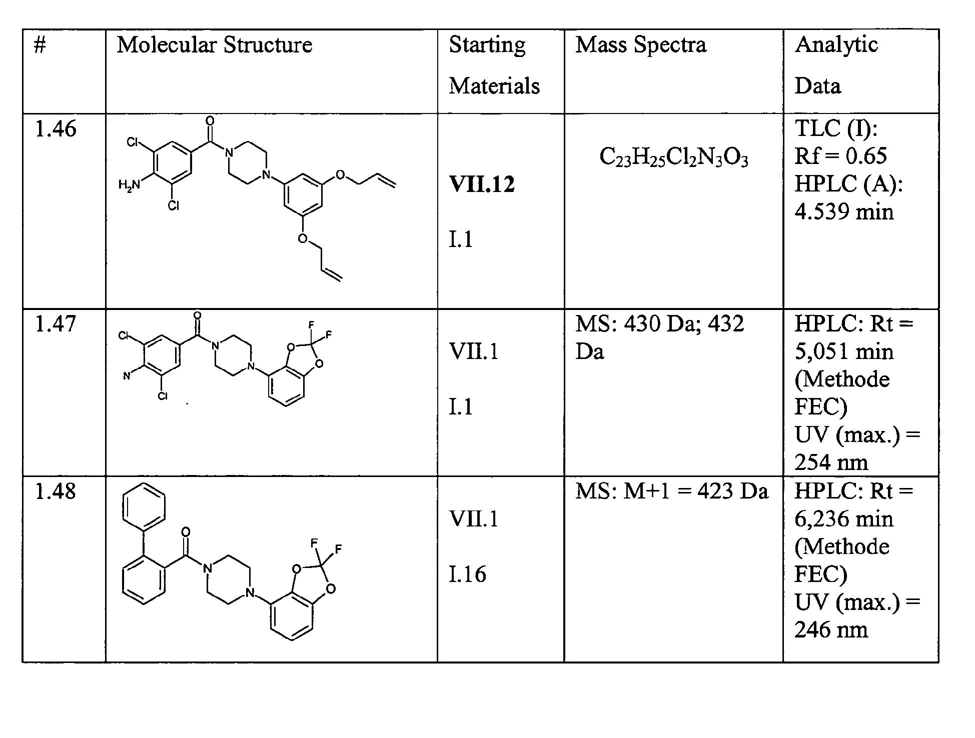

### Analytical Data:

TLC methods (method and Rf are indicated):
(A): Silica; petrol ether / ethyl acetate (5:1)
(B): Silica; DCM / methanol (9: 1)
(C): Silica; petrol ether / ethyl acetate (3:2)
(D): Silica; DCM / methanol / aq. ammonia (90:10:1)
(E): Silica; DCM / methanol (50:1)
(F): Silica; DCM / methanol / aq. ammonia (80:20:1)
(G): Silica; DCM / methanol (80:2)
(H): Silica; DCM / methanol (15:1)
(I): Silica; DCM / methanol (95:5)
(K): Silica; DCM / methanol (99:1)
(L): Silica; DCM / methanol (98:2)
(M): Silica; DCM

HPLC methods (method and retention time are indicated):
(A): Equipment: Agilent 1100 Series; MS: 1100 Series LC/MSD (API-ES(+/- 3000V,
   Quadrupol, G1946D), Mode: Scan pos 100-1000, neg 100-1000; column: Waters; Part No.186000594; Xterra MS C18 2.5µm; 2.1x50mm column
   Solvent: A: H₂O demin. with 0, 1 % HCOOH, B: acetonitrile HPLC grade with 0, 1 % HCOOH
   Detection: peakwide > 0,1min (2s); 190-450 nm, UV 254 nm (bandwide 8, reference off), UV 230 nm (bandwide 8, reference off)
   Injection: 3 *µ*l standard injection
   Flow: 0.6 ml/min; column temp.: 35°C
   Pump gradient: 0.0-0.5 min 5 % solvent B; 0.5-1.5 min 5 % -> 50 % solvent B; 1.5-4.0 min 50 % -> 95 % solvent B; 4.0-6.0 min 95 % solvent B; 6.0-6.5 min 95 % -> 5 % solvent B; 1.5 min post run 5 % solvent B
(B): Equipment: Waters ZMD, Alliance 2690 HPLC, Waters 2700 Autosampler, Waters 996 Detector; MS API-ES (25V Cone, -3kV Capillary, Quadrupol, New ZMD SN:MC198) Mode: Scan pos 140-850; column: Waters X-Terra™ MS C18 3.5 µM, 4.6 mm x 50 mm;
   Solvent: A: H₂O demin. with 0.1% TFA, B: acetonitrile with 0.1% TFA
   Detection: 210-500 nm,
   Flow: 1.0 ml/min; column temp.: 25 °C
   Pump gradient: 0.0-0.1 min 5 % solvent B; 0.1-5.1 min 5 % -> 98 % solvent B; 5.1-7.5 min 98 % solvent B; 7.5-8.0 min 5 % solvent B

### Example 2.1 1

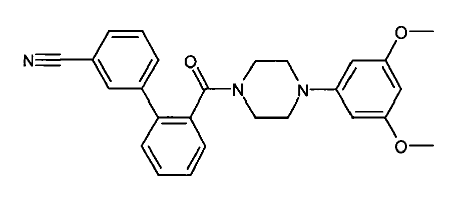

To a solution of 1-(2-iodobenzoyl)-4-(3,5-dimethoxyphenyl)-piperazine (compound VIII.1; 1 g; 2.21 mmol) in 25 ml dioxane under argon atmosphere are subsequently added 4.5 ml of a 2 M aqueous sodium carbonate (9 mmol), tetrakis(triphenylphosphine)-palladium (120 mg; 0.104 mmol), and 3-cyanophenylboronic acid (compound V.5; 400 mg; 2.72 mmol). The reaction mixture is refluxed for 15 h. After solvent evaporation, the crude product is purified by reversed phase column chromatography (RP-silica, eluent: gradient starting with acetonitrile/water 5:95, reaching 98:2 within 35 min).
Yield: 0.746 g (79 %; brownish solid)
¹H-NMR (DMSO) d = 3.30 (br s, 4H), 3.67 (br s, 4H), 5.96 (s, 2H), 5.97-5.98 (m, 1H), 7.41-7.43 (m, 1H), 7.54-7.56 (m, 1H), 7.55-7.59 (m, 2H), 7.65-7.68 (m, 1H), 7.74-7.76 (m, 1H), 7.84-7.86 (m, 1H), 7.86 (s, 1H).
Rf value TLC: 0.66 (silica; DCM/methanol 95:5)
Melting point: 67-70°C
Mass Spectrum: m/z = 428 [M+H]⁺ (C₂₆H₂₅N₃O₃)
HPLC retention time: 4.156 min (Method A)

The following compounds are prepared analogously to the procedure as described for example 2.1 using the starting materials given in the table.

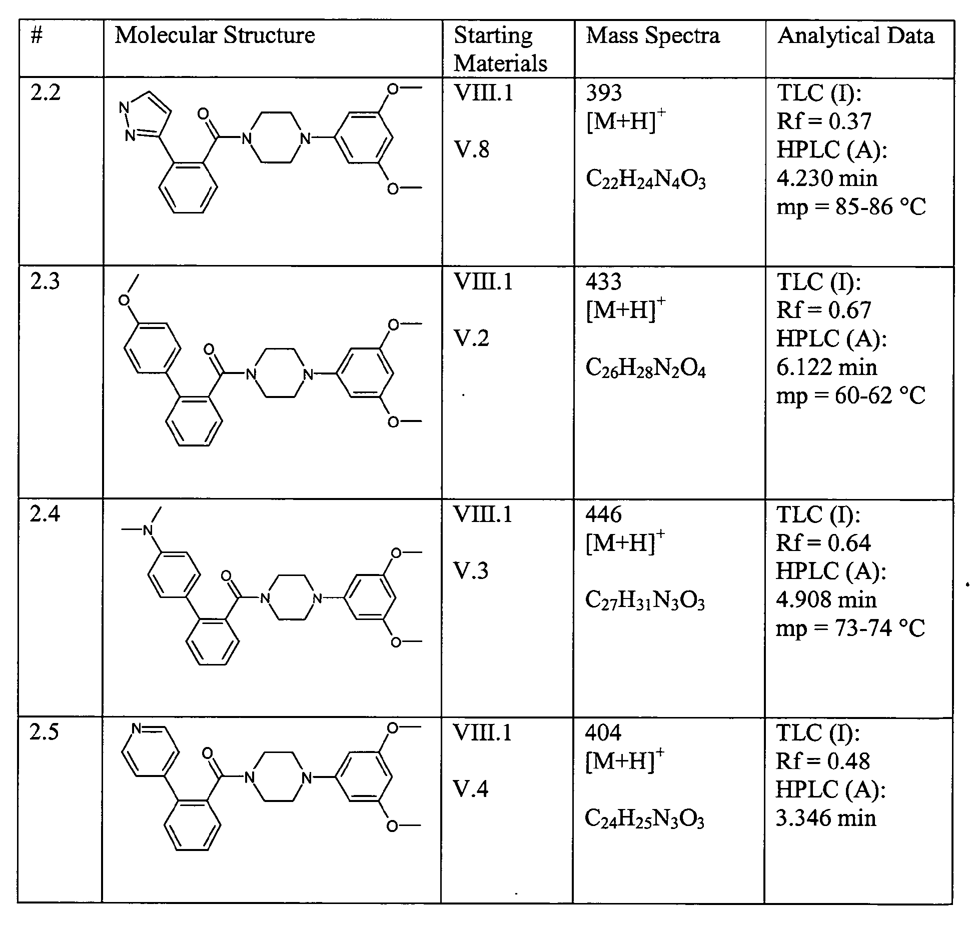

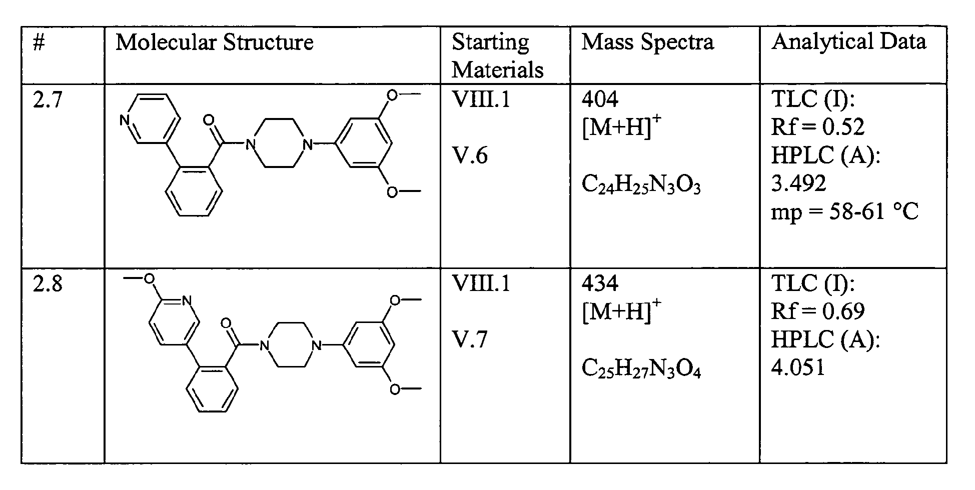

### Example 3.1 and Example 3.2

To a solution of example compound 1.15 (350 mg; 0.853 mmol) in 5 ml DMF under argon atmosphere is added sodium hydride (60% in mineral oil; 75.1 mg; 1.88 mmol). After several minutes of stirring, methyl iodide (0.117 ml; 1.88 mmol) is added. The reaction mixture is stirred overnight at ambient temperature, then quenched with aqueous ammonia and again stirred for several minutes. The solvent is evaporated and the desired products are isolated from mixture by reversed phase column chromatography (RP-silica, eluent: acetonitrile/water gradient).

Examples 3.3 / 3.4 are prepared analogously to the procedure as described for examples 3.1 / 3.2 using 7.8 as starting material.

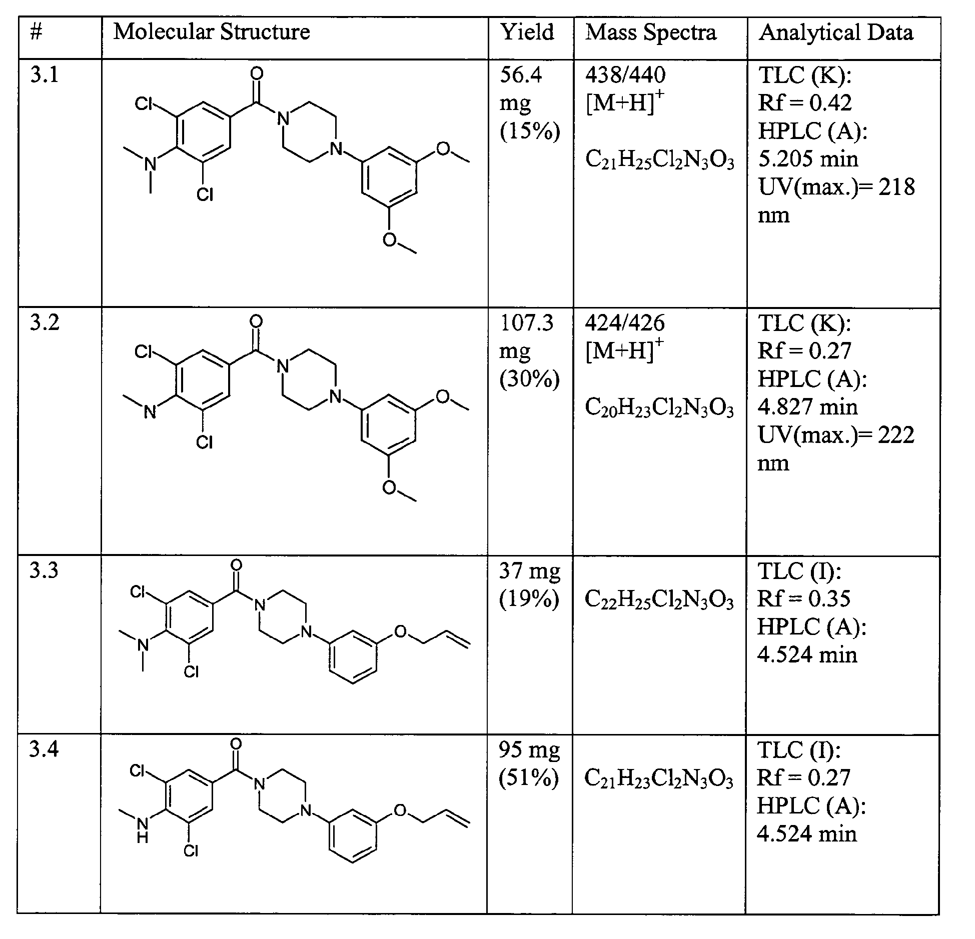

### Example 4.1

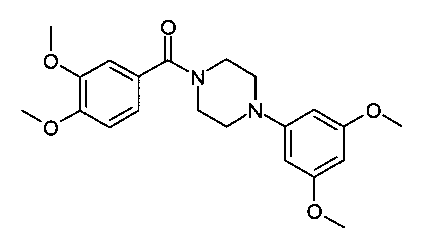

To a solution of DMSO (0.2 ml) containing 3,4-dimethoxy-benzoic acid (compound 1.9; 3.64 mg; 0.02 mmol) and ethyl diisopropyl amine (0.02 mmol) in a microtiter plate is added a DMSO solution (0.1 ml) of PFTU (8.56 mg; 0.02 mmol). The plate is shaken for 5 min, then a DMSO solution (0.2 ml) containing 1-(3,5-Dimethoxyphenyl)-piperazine (compound II.9; 4.67 mg; 0.021 mmol) and ethyl diisopropyl amine (0.02 mmol) is added.
The plate is shaken for further 5 min, kept overnight at ambient temperature, then the solvent is evaporated. The solid residue is checked for purity (which is 91% by HPLC-MS) and tested without further purification.
Mass Spectrum: m/z = 387 [M+H]⁺ (C₂₁H₂₆N₂O₅)
HPLC retention time: 4.11 min (method B)

The following compounds are prepared analogously to the procedure as described for example 2.1 using the starting materials given in the table.

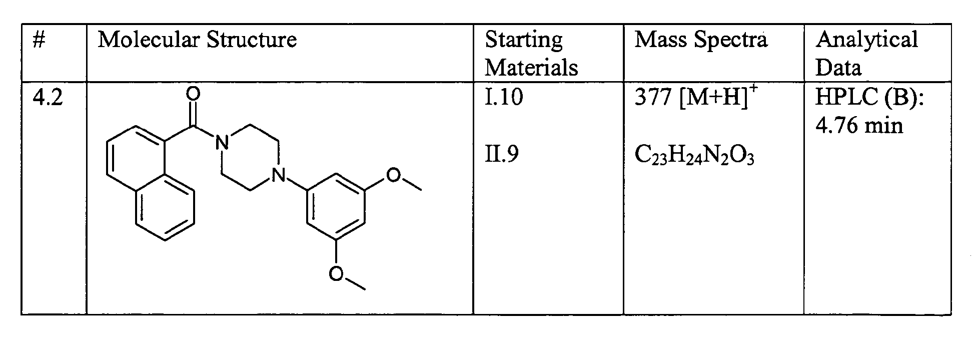

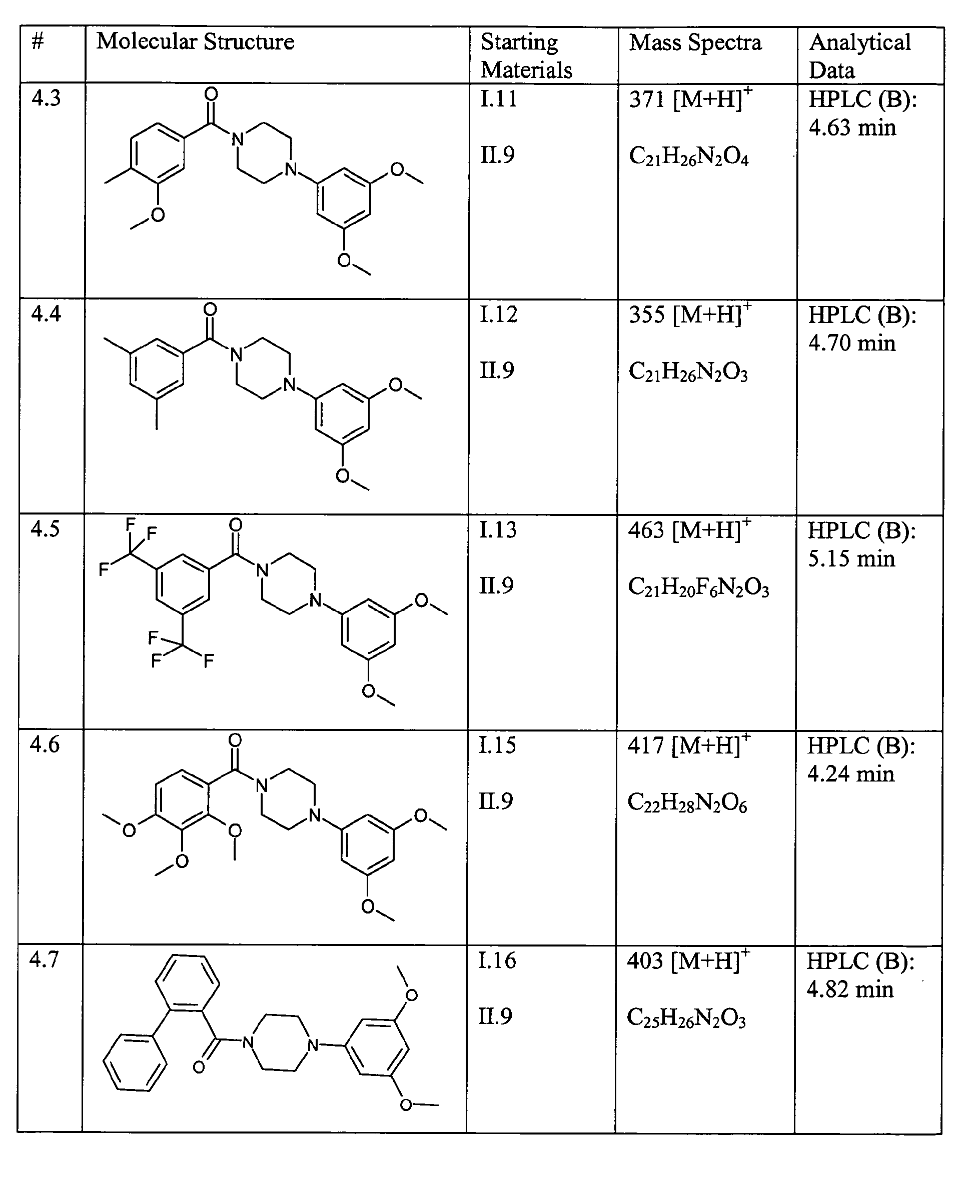

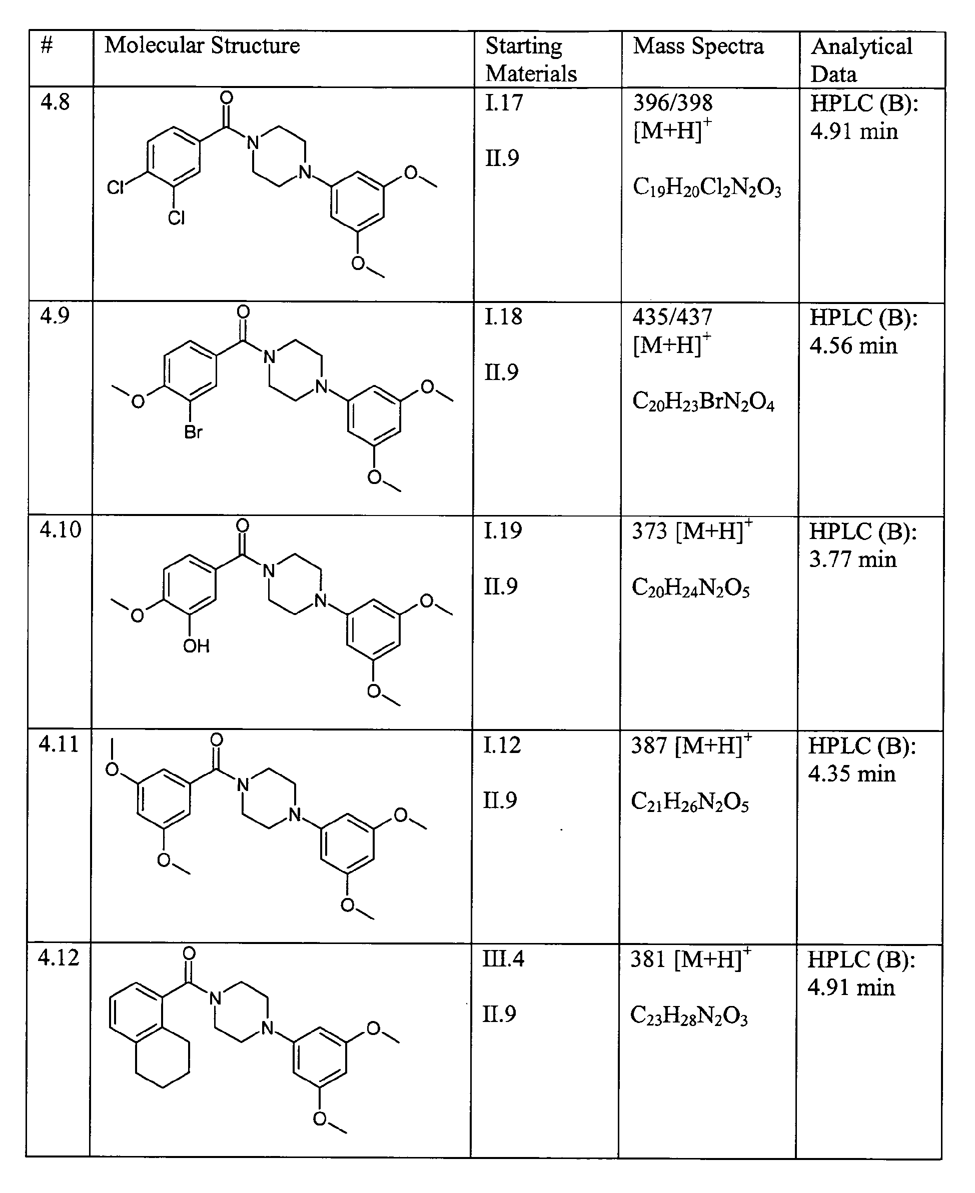

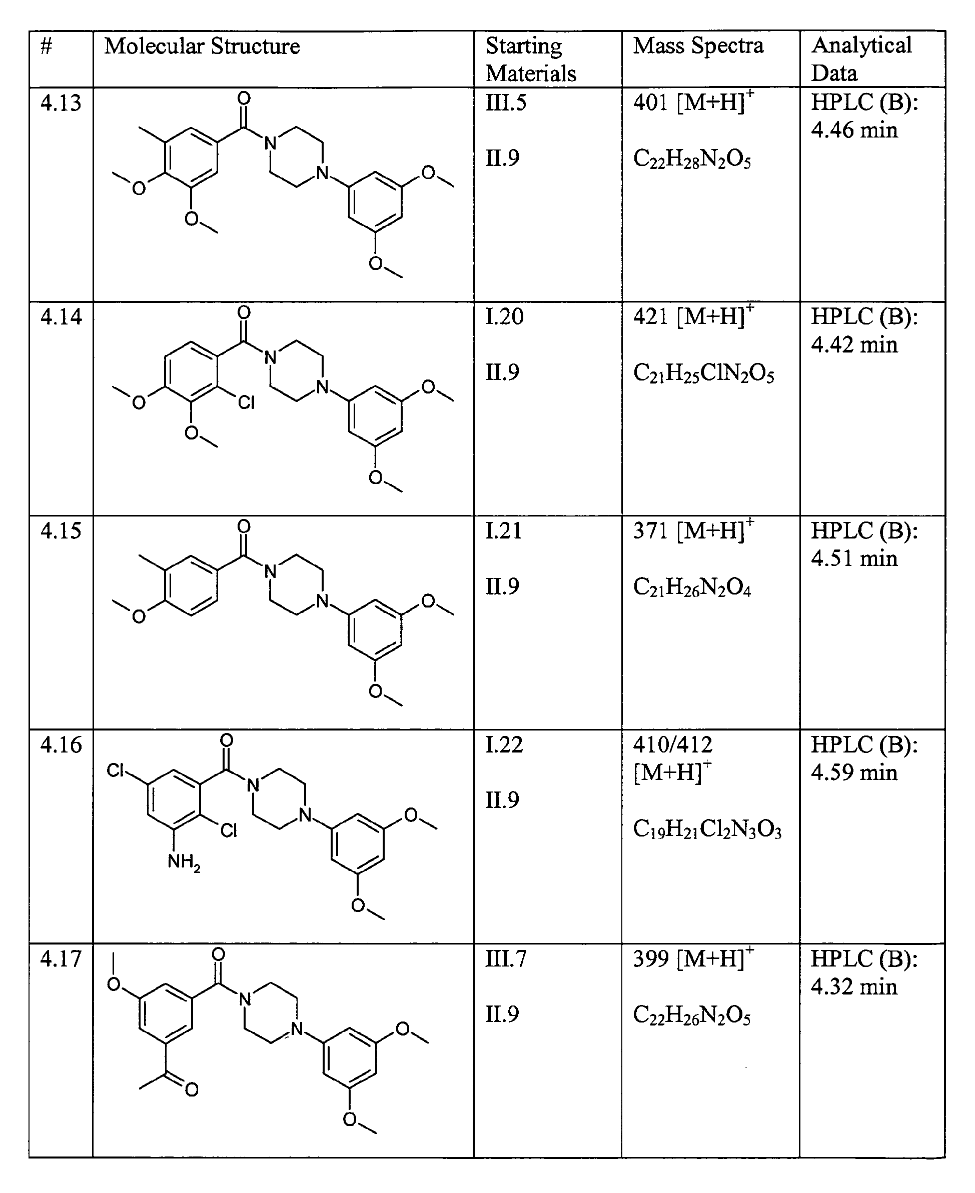

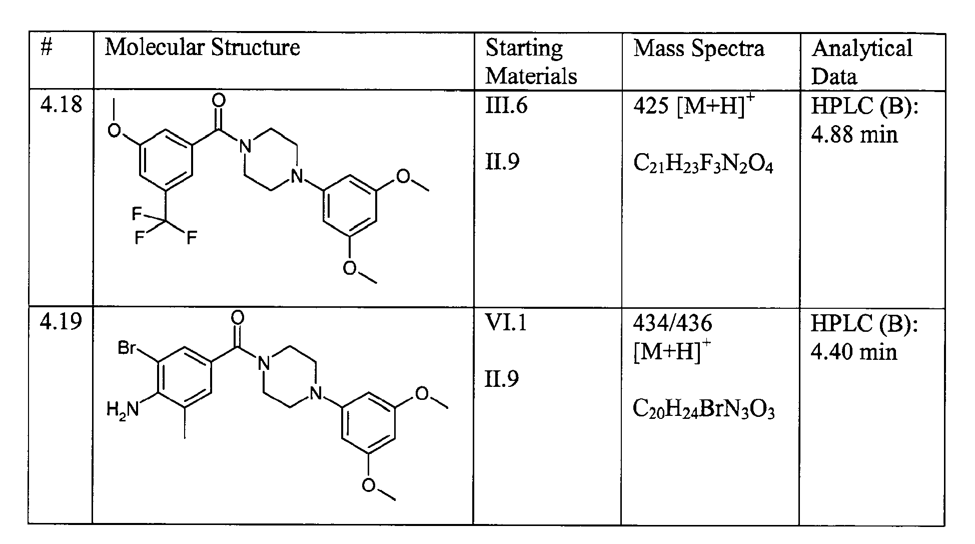

### Example 5.1

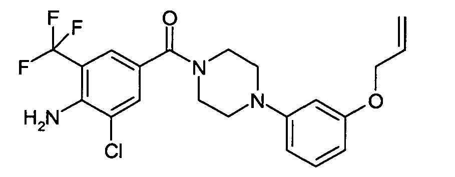

To a solution of compound 1.18 (0.4 g; 1 mmol) in acetone (5 ml) are added allyl bromide (0.242 g; 2 mmol) and potassium carbonate (0.276 g; 2 mmol). The reaction mixture is
stirred at 50°C for 4 hours, then additional allyl bromide and potassium carbonate (2 mmol each) are added and the reaction mixture is stirred at 50°C overnight. The solvent is evaporated. The residue is taken up in water and extracted with ethyl acetate. The organic layer is dried over anhydrous sodium sulphate and concentrated in vacuo to yield the desired compound as oil.
Yield: 0.436 g (99 %)
¹H-NMR (DMSO) d = 3.18 (br s, 4H), 3.62 (br s, 4H), 4.54 (d, 2H), 5.25 (d, 1H), 5.39 (d, 1H), 5.99-6.09 (m, 1H), 6.12 (br s, 2H), 6.38-6.44 (m, 1H), 6.48-6.58 (m, 2H), 7.08-7.16 (m, 1H), 7.49 (s, 1H), 7.66 (s, 1H).
Rf value TLC: 0.7 (Silica; DCM / methanol (80:2))
Mass Spectrum: m/z = 440/442 [M+H]⁺ (C₂₁H₂₁ClF₃N₃O₂)

Following compounds are prepared analogously to the procedure as described for example 5.1 using the starting materials given in the table.

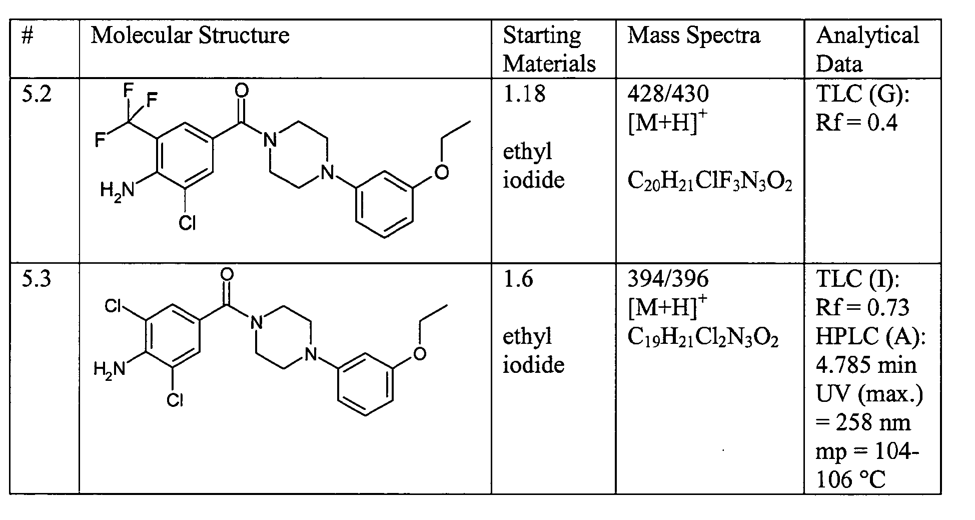

### Example 6.1

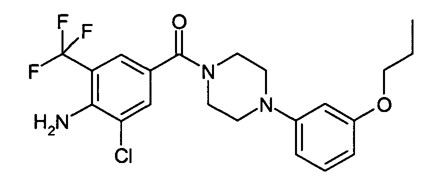

Compound 5.1 (0.1 g; 0.227 mmol) in methanol (5 ml) is hydrogenated in a Parr apparatus (0.01 g palladium on charcoal (10%); 50 psi hydrogen pressure) until hydrogen uptake comes to an end. The catalyst is filtered off. The solvent is evaporated and the residue is purified by column chromatography (silica, eluent: DCM/Methanol 90: 1).
Yield: 0.040 g (40 %; colourless viscous oil)
¹H-NMR(DMSO) d = 0.97 (t, 3H), 1.65-1.77 (m, 2H), 3.17 (br s, 4H), 3.63 (br s, 4H), 3.89 (t, 2H), 6.11 (br s, 2H), 6.37-6.40 (m, 1H), 6.45-6.47 (m, 1H), 6.50-6.53 (m, 1H), 7.08-7.13 (m, 1H), 7.48 (s, 1H), 7.65 (s, 1H).
Rf value TLC: 0.9 (Silica; DCM/methanol 9:1)
Mass Spectrum: m/z = 442/444 [M+H]⁺ (C₂₁H₂₃ClF₃N₃O₂)

### Example 7.1

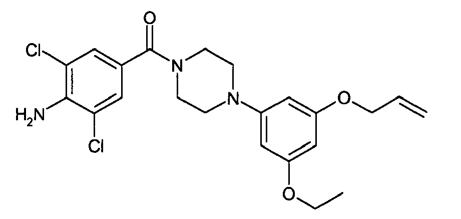

To a solution of compound 1.42 (80 mg, 0.19 mmol) in DMF (2 ml) are added ethyl bromide (16 *µ*L, 0.21 mmol) and potassium carbonate (35 mg, 0.25 mmol). The reaction mixture is stirred overnight at ambient temperature, further ethyl bromide (16 *µ*l, 0.21 mmol) is added and the reaction mixture is stirred for 4 hours. After solvent evaporation, the residue is purified by reversed phase column chromatography (RP-silica, eluent: acetonitrile/water gradient).
Yield: 0.041 g (48 %; colourless viscous oil) C₂₂H₂₅Cl₂N₃O₃
¹H-NMR(DMSO) d = 1.28 (t, 3H), 3.15 (br s, 4H), 3.58 (br s, 4H), 3.96 (q, 2H), 4.49-4.51 (m, 2H), 5.22-5.24 (m, 1H), 5.35-5.39 (m, 1H), 5.91 (br s, 2H), 5.98-5.99 (m, 1H), 6.06-6.07 (m, 1H), 6.09-6.10 (m, 1H), 7.34 (s, 2H).
Rf value TLC: 0.57 (Silica; DCM/methanol 95:5)
HPLC (Method A): 4.476 min
Following compounds are prepared analogously to the procedure as described for example 7.1 using the starting materials given in the table.

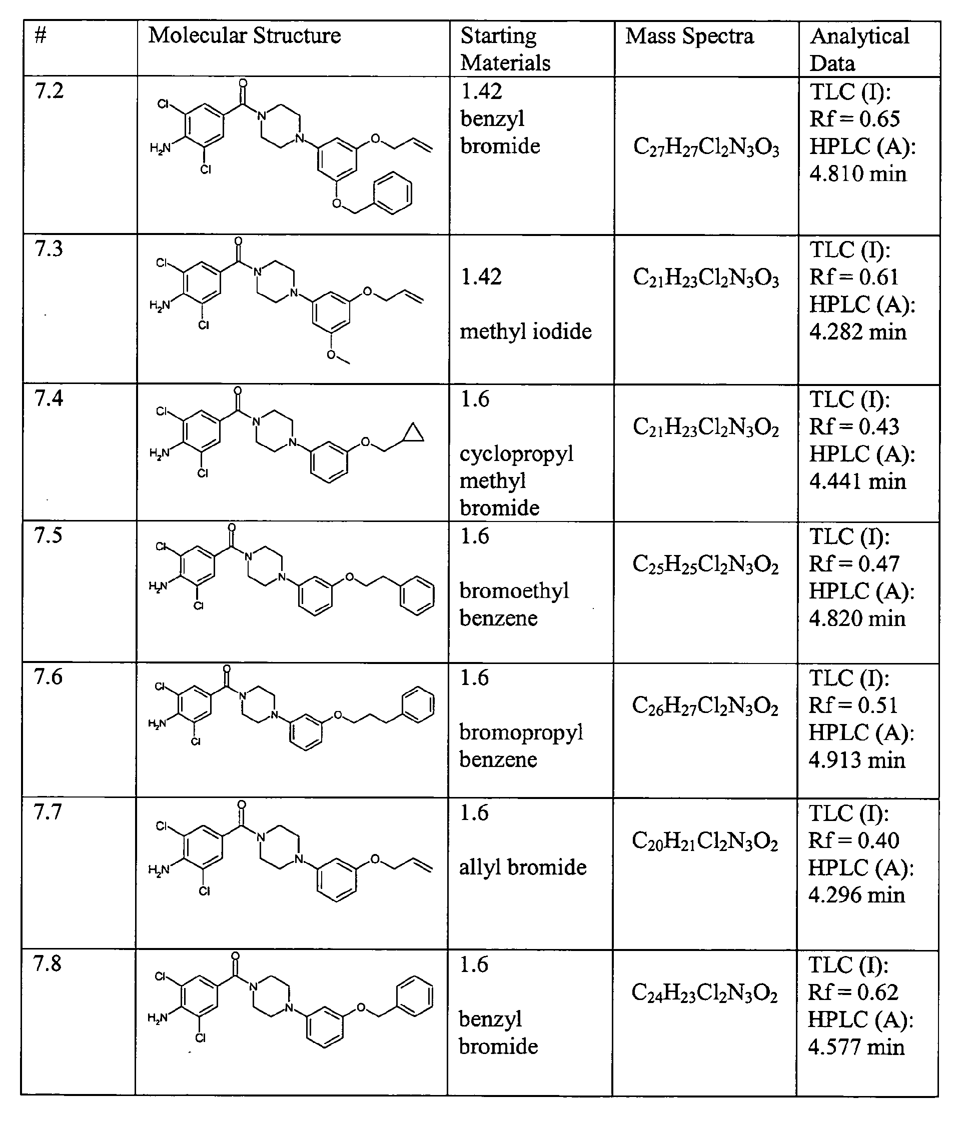

### Biological experiments

The compounds of the invention are useful in binding to tubulin and thereby inhibiting the activity of tubulin. In doing so, these compounds are useful in blocking disease processes by binding to tubulin. Accordingly, the compounds of the present invention are useful in treating cancer or other abnormal proliferative diseases. Cancers are classified in two ways: by the type of tissue in which the cancer originates (histological type) and by primary site, or the location in the body where the cancer first developed. The most common sites in which cancer develops include the skin, lungs, female breasts, prostate, colon and rectum, cervix and uterus.

The compounds are thus useful in the treatment of a variety of cancers, including but not limited to the following:
- AIDS-related cancer such as Kaposi's sarcoma;
- bone related cancer such as Ewing's family of tumors and osteosarcoma;
- brain related cancer such as adult brain tumor, childhood brain stem glioma, childhood cerebellar astrocytoma, childhood cerebral astrocytoma/malignant glioma, childhood ependymoma, childhood medulloblastoma, childhood supratentorial primitive neuroectodermal tumors, childhood visual pathway and hypothalamic glioma and other childhood brain tumors;
- breast cancer;
- digestive/gastrointestinal related cancer such as anal cancer, extrahepatic bile duct cancer, gastrointestinal carcinoid tumor, colon cancer, esophageal cancer, gallbladder cancer, adult primary liver cancer, childhood liver cancer, pancreatic cancer, rectal cancer, small intestine cancer and stomach (gastric) cancer;
- endocrine related cancer such as adrenocortical arcinoma, gastrointestinal carcinoid tumor, islet cell carcinoma (endocrine pancreas), parathyroid cancer, pheochromocytoma, pituitary tumor and thyroid cancer;
- eye related cancer such as intraocular melanoma, and retinoblastoma;
- genitourinary related cancer such as bladder cancer, kidney (renal cell) cancer, penile cancer, prostate cancer, transitional cell renal pelvis and ureter cancer, testicular cancer, urethral cancer, Wilms' tumor and other childhood kidney tumors;
- germ cell related cancer such as childhood extracranial germ cell tumor, extragonadal germ cell tumor, ovarian germ cell tumor and testicular cancer;
- gynecologic related cancer such as cervical cancer, endometrial cancer, gestational trophoblastic tumor, ovarian epithelial cancer, ovarian germ cell tumor, ovarian low malignant potential tumor, uterine sarcoma, vaginal cancer and vulvar cancer;
- head and neck related cancer such as hypopharyngeal cancer, laryngeal cancer, lip and oral cavity cancer, metastatic squamous neck cancer with occult primary, nasopharyngeal cancer, oropharyngeal cancer, paranasal sinus and nasal cavity cancer, parathyroid cancer and salivary gland cancer;
- hematologic/blood related cancer such as leukemias, such as adult acute lymphoblastic leukemia, childhood acute lymphoblastic leukemia, adult acute myeloid leukemia, childhood acute myeloid leukemia, chronic lymphocytic leukemia, chronic myelogenous leukemia and hairy cell leukemia; and lymphomas, such as AIDS-related lymphoma, cutaneous T-cell lymphoma, adult Hodgkin's lymphoma, childhood Hodgkin's lymphoma, Hodgkin's lymphoma during pregnancy, mycosis fungoides, adult non-Hodgkin's lymphoma, childhood non-Hodgkin's lymphoma, non-Hodgkin's lymphoma during pregnancy, primary central nervous system lymphoma, Sezary syndrome, cutaneous T-cell lymphoma and Waldenström's macroglobulinemia and other hematologic/blood related cancer such as chronic myeloproliferative disorders, multiple myeloma/plasma cell neoplasm, myelodysplastic syndromes and myelodysplastic/myeloproliferative diseases;
- lung related cancer such as non-small cell lung cancer and small cell lung cancer
- musculoskeletal related cancer such as Ewing's family of tumors, osteosarcoma, malignant fibrous histiocytoma of bone, childhood rhabdomyosarcoma, adult soft tissue sarcoma, childhood soft tissue sarcoma and uterine sarcoma;
- neurologic related cancer such as adult brain tumor, childhood brain tumor, brain stem glioma, cerebellar astrocytoma, cerebral astrocytoma/malignant glioma, ependmoma, medulloblastoma, supratentorial primitive neuroectodermal tumors, visual pathway and hypothalamic glioma and other brain tumors such as neuroblastoma, pituitary tumor and primary central nervous system lymphoma;
- respiratory/thoracic related cancer such as non-small cell lung cancer, small cell lung cancer, malignant mesothelioma, thymoma and thymic carcinoma;
- skin related cancer such as cutaneous T-cell lymphoma, Kaposi's sarcoma, melanoma, Merkel cell carcinoma and skin cancer

Compounds binding to tubulin may also inhibit angiogenesis and affect abnormal cellular proliferation and can be used to treat certain forms of blindness related to retinal vascularization, arthritis, especially inflammatory arthritis, multiple sclerosis, restenosis and psoriasis and may induce apoptosis, a physiological cell death process critical for normal development and homeostasis.

The compounds of the invention are also useful for treatment of e.g. follicular lymphomas, carcinomas with p53 mutations, hormone dependent tumor of the breast, prostate and ovary and precancerous lesions such as familial adenomatous polyposis, viral infections, autoimmune diseases such as systemic lupus erythematosus, immune mediated glomerulonephritis, rheumatoid arthritis, psoriasis, inflammatory bowel diseases and autoimmune diabetes mellitus.

The compounds of the invention can be used in combination with known anticancer and cytotoxic agents and treatments including radiation, especially where the second drug acts in a different phase of the cell cycle.

### Methods

The in vitro assessment of the biological activity of the inventive compounds is performed as follows:

### In vitro tubulin polymerization assay (TPA)

The assay is performed according to Bollag MD et al. (Epothilones, a new class of microtubule-stabilizing agents with a taxol-like mechanism of action. Cancer Research 55: 2325-2333, 1995). Tubulin heterodimers (1.6 mg/ml; 160 *µ*g/lassay), from bovine brain (Cytoskeleton), are incubated with test compounds (10 µM final concentration) in PEM (100 mM PIPES, 1 mM EGTA, and 1 mM MgCl₂) buffer (pH 6.6) containing 1 mM GTP in a total volume of 100 µl at 37°C for 1 h. Samples (80 µl) are then transferred to a 96-well Millipore Multiscreen Durapore hydrophilic 0.22-µm pore size filtration plate. Microtubules are recovered on the filters and are stained with 50 µl of Amido Black solution [0.1% w/v napthol blue black (Sigma), 45% v/v methanol, and 10% v/v acetic acid] for 2 min. Vacuum is applied, and unbound dye is removed by two additions of 200 µl of destain solution (90% v/v methanol, 2% v/v acetic acid). The microtubule bound dye is eluted by incubation with 200 µl of elution solution (25 mM NaOH, 0.05 mM EDTA, and 50% v/v ethanol) for 20 min. Next, 150 µl of elution solution is transferred to a 96-well half area plate, and the absorbance is measured at 600 nm using the Wallac Victor Multilabel counter (Perkin-Elmer/Wallac, Freiburg, Germany). The assay format allows the identification of novel tubulin ligands and gives some indication as to their mechanism of action (e.g. microtubule stabilizer or destabilizer). A result of less than 50% indicates inhibition of tubulin polymerization (destabilizer). A result above 150% indicates induction of tubulin polymerization (stabilizer).

Most of the compounds have values below 50% and are therefore destabilizers.

### In vitro cytotoxicity assay (MTS)

Cytotoxicity is assessed in HeLa human squamous cell carcinoma by MTS (3- (4, 5-dimethylthiazol-2-yl) -5- (3- carboxymethoxyphenyl) -2- (4- sulfenyl) -2*H* tetrazolium, inner salt) assay as reported in T.L.Riss, et al., "Comparison of MTT, XTT and a novel tetrazolium compound MTS for in vitro proliferation and chemosensitivity assays" Mol. Biol. Cell 3 (Suppl.): 184a, 1992).
Cells are plated at 2500 cells/well in 96 well microtiter plates and 24 hours later drugs are added and serial diluted (10*µ*M starting concentration). The cells are incubated at 37° for 4-5 days at which time the tetrazolium dye, MTS at 333*µ*g/ml (final concentration), in combination with the electron coupling agent phenazine methosulfate at 25*µ*M (final concentration) is added. The cells are then incubated for 2-3 hours at 37°. The assay is based on the cleavage of the tetrazolium compound MTS to colored formazan by the "succinate-tetrazolium reductase" mitochondrial enzyme, active only in living (metabolic active) cells. The presence of the electron coupling reagent PMS allows the formation of a stable solution. The amount of dye is quantitated spectrophotometrically at 492nM. The absorbance is a function of the concentration of converted dye and directly correlates to the number of metabolically active (living) cells in the culture. The results are expressed as an IC50, which is the drug concentration required to inhibit cell proliferation to 50% of that of untreated control cells.

The IC50 values for compounds of this invention fall below 10 *µ*M.

The compounds according to the invention may be administered by oral, transdermal or parenteral route or by inhalation. The compounds according to the invention are present as active ingredients in conventional preparations, e.g. in compositions consisting essentially of an inert pharmaceutical carrier and an effective dose of the active substance, such as for example plain or coated tablets, capsules, lozenges, powders, solutions, suspensions, emulsions, syrups, suppositories, transdermal systems, etc. An effective dose of the compounds according to the invention is between 1 and 100, preferably between 1 and 50, most preferably between 5-30 mg/dose, for oral administration, and between 0.001 and 50, preferably between 0.1 and 10 mg/dose for intravenous or intramuscular administration. For inhalation, solutions containing 0.01 to 1.0, preferably 0.1 to 0.5% of active substance are suitable according to the invention. For inhalation, the use of powders is preferred. It is also possible to use the compounds according to the invention as a solution for infusion, preferably in physiological saline or nutrient salt solution.

The compounds according to the invention may be used on their own or in conjunction with other active substances according to the invention, optionally also in conjunction with other pharmacologically active substances. Suitable preparations include for example tablets, capsules, suppositories, solutions, elixirs, emulsions or dispersible powders. Suitable tablets may be obtained, for example, by mixing the active substance(s) with known excipients, for example inert diluents such as calcium carbonate, calcium phosphate or lactose, disintegrants such as corn starch or alginic acid, binders such as starch or gelatine, lubricants such as magnesium stearate or talc and/or agents for delaying release, such as carboxymethyl cellulose, cellulose acetate phthalate, or polyvinyl acetate. The tablets may also comprise several layers.

Coated tablets may be prepared accordingly by coating cores produced analogously to the tablets with substances normally used for tablet coatings, for example collidone or shellac, gum arabic, talc, titanium dioxide or sugar. To achieve delayed release or prevent incompatibilities the core may also consist of a number of layers. Similarly the tablet coating may consist of a number of layers to achieve delayed release, possibly using the excipients mentioned above for the tablets.

Syrups or elixirs containing the active substances or combinations thereof according to the invention may additionally contain a sweetener such as saccharine, cyclamate, glycerol or sugar and a flavor enhancer, e.g. a flavoring such as vanillin or orange extract. They may also contain suspension adjuvants or thickeners such as sodium carboxymethyl cellulose, wetting agents such as, for example, condensation products of fatty alcohols with ethylene oxide, or preservatives such as p-hydroxybenzoates.

Solutions for injection and infusion are prepared in the usual way, e.g. with the addition of preservatives such as p-hydroxybenzoates, or stabilizers such as alkali metal salt of ethylenediamine tetra-acetic acid, and transferred into injection vials or ampoules.

Capsules containing one or more active substances or combinations of active substances may for example be prepared by mixing the active substances with inert carriers such as lactose or sorbitol and packing them into gelatine capsules.

Suitable suppositories may be made for example by mixing with carriers provided for this purpose, such as neutral fats or polyethyleneglycol or the derivatives thereof.

A therapeutically effective daily dose is between 1 and 800 mg, preferably 10-300 mg, in adults.

The Examples that follow illustrate the present invention without, however, restricting its scope.

### Examples of Pharmaceutical Formulations

| Tablets | per tablet |
|---|---|
| Active substance | 100 mg |
| Lactose | 140 mg |
| Corn starch | 240 mg |
| Polyvinylpyrrolidone | 15 mg |
| Magnesium stearate | 5 mg |
| | 500 mg |

The finely ground active substance, lactose and some of the corn starch are mixed together. The mixture is screened, moistened with a solution of polyvinylpyrrolidone in water, kneaded, wet-granulated and dried. The granules, the remaining corn starch and the magnesium stearate are screened and mixed together. The mixture is compressed to produce tablets of suitable shape and size.

| Tablets | per tablet |
|---|---|
| Active substance | 80 mg |
| Lactose | 55 mg |
| Corn starch | 190 mg |
| Microcrystalline cellulose | 35 mg |
| Polyvinylpyrrolidone | 15 mg |
| Sodium-carboxymethyl starch | 23 mg |
| Magnesium stearate | 2 mg |
| | 400 mg |

The finely ground active substance, some of the corn starch, lactose, microcrystalline cellulose and polyvinylpyrrolidone are mixed together, the mixture is screened and worked with the remaining corn starch and water to form a granulate which is dried and screened. The sodium-carboxymethyl starch and the magnesium stearate are added and mixed in and the mixture is compressed to form tablets of a suitable size.

| Coated tablets | per coated tablet |
|---|---|
| Active substance | 5 mg |
| Corn starch | 41.5 mg |
| Lactose | 30 mg |
| Polyvinylpyrrolidone | 3 mg |
| Magnesium stearate | 0.5 mg |
| | 80 mg |

The active substance, corn starch, lactose and polyvinylpyrrolidone are thoroughly mixed and moistened with water. The moist mass is pushed through a screen with a 1 mm mesh size, dried at about 45°C. and the granules are then passed through the same screen. After the magnesium stearate has been mixed in, convex tablet cores with a diameter of 6 mm are compressed in a tablet-making machine. The tablet cores thus produced are coated in known manner with a covering consisting essentially of sugar and talc. The finished coated tablets are polished with wax.

| Capsules | per capsule |
|---|---|
| Active substance | 50 mg |
| Corn starch . | 268.5 mg |
| Magnesium stearate | 1.5 mg |
| | 320 mg |

The substance and corn starch are mixed and moistened with water. The moist mass is screened and dried. The dry granules are screened and mixed with magnesium stearate.
The finished mixture is packed into size 1 hard gelatine capsules.

Ampoule solution active substance 50 mg sodium chloride 50 mg water for inj. 5 ml

The active substance is dissolved in water at its own pH or optionally at pH 5.5 to 6.5 and sodium chloride is added to make it isotonic. The solution obtained is filtered free from pyrogens and the filtrate is transferred under aseptic conditions into ampoules which are then sterilized and sealed by fusion. The ampoules contain 5 mg, 25 mg and 50 mg of active substance.

| Suppositories | |
|---|---|
| Active substance | 50 mg |
| Solid fat | 1650 mg |
| | 1700 mg |

The hard fat is melted. At 40°C the ground active substance is homogeneously dispersed. It is cooled to 38°C and poured into slightly chilled suppository moulds.

### List of Abbreviations

AcOH ― Acetic acid
boc ― *tert*. Butyl-oxycarbonyl
Ctrl Control
decomp. ― decomposition
demin. ― demineralized
DBU ― Diaza(1,3)bicyclo[5.4.0]undecane
DIPEA ― Diisopropylethyl amine
DMF ― *N,N-*Dimethylformamide
EGTA ― Ethylene glycol-bis-(2-aminoethyl)-*N*,*N*,*N'*,*N*'-tetraacetic acid
EtOAc ― Ethyl acetate
GTP ― Guanidine triphosphate
HPLC ― High performance liquid chromatography
LC/MS ― Liquid chromatography mass spectrometer
MS ― Mass spectrometer
MTS ― 3-(4, 5-Dimethylthiazol-2-yl)-5-(3-carboxymethoxyphenyl)-2-(4-sulfenyl)-2H tetrazolium, inner salt
n.d. ― not determined
NMR ― Nuclear Magnetic Resonance
NP-HPLC ― Normal Phase high performance liquid chromatography
PIPES ― Piperazine-*N,N'*-bis(2-ethanesulfonic acid)
PFTU ― *o*-Pentafluorophenyl-tetramethyluronium tetrafluoroborate
PMS ― *N*-Methyldibenzopyrazine methyl sulfate salt
RP-HPLC ― Reversed phase high performance liquid chromatography
RP-MPLC ― Reverse phase middle pressure liquid chromatography
RP-silica ― Reversed phase silica
rt ― Retention time
RT ― Room temperature
TBTU - O-(Benzotriazol-1-yl)-*N*,*N*,*N*,*N*'-tetramethyluronium tetrafluoroborate
TFA ― Trifluoroacetic acid
THF ― Tetrahydrofuran
TLC - thin layer chromatography
TPA - Tubulin Polymerisation Assay
UV ― Ultraviolet

## Claims

1. A compound of formula (I) 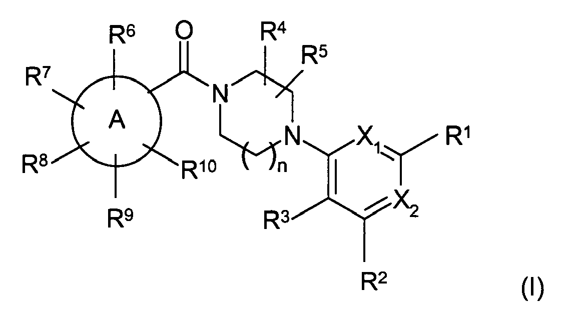 of its salts or pharmaceutically acceptable derivatives thereof, wherein
A represents mono- or bicyclic carbocyclic aryl; and
**R**¹ and **R**² are independently selected from the group consisting of hydrogen, halogen and cyano or from an optionally substituted group consisting of C₁₋₆alkyl-, R^{a}O-, R^{a}R^{b}N-, C₁₋₆alkyl-C(=O)-, C₁₋₆alkyl-C(=O)-(R^{b})-, C₁₋₆alkyl-N(R^{b})-C(=O)- and R^{a}O-C₁₋₆alkyl-, and
**R**^{**3**} is selected from the group consisting of hydrogen, halogen, cyano, C₁₋₆alkyl- and R^{a}O-or
**R**^{**2**} and **R**^{**3**} may combine to form an optionally substituted cyclic group -O-(CH₂)ₚ-O-; and
**R**^{**4**} and **R**^{**5**} are independently selected from hydrogen and C₁₋₆alkyl-; and
**R**^{**6**}**, R**^{**7**}**, R**^{**8**}**, R**^{**9**} and **R**^{**10**} are independently selected from the group consisting of hydrogen, halogen, nitro and cyano or from an optionally substituted group consisting of C₁₋₆alkyl-, R^{a}R^{b}N-, R^{a}O-, R^{a}-C(=O)-N(R^{b})-, R^{a}R^{b}N-C(=O)-, R^{a}R^{b}N-SO₂-, R^{a}-SO₂-N(R^{b})-, R^{a}-C(=O)-, R^{a}-SO₂-, R^{a}-SO-, R^{a}-S-, R^{a}R^{b}-C₁₋₆alkyl-, R^{a}R^{b}N-C₁₋₆alkyl-O-, R^{a}O-C₁₋₆alkyl- and aryl; and
**R**^{**a**} and **R**^{**b**} are independently selected from hydrogen, C₁₋₆alkyl-, cycloalkyl-, aryl-C ₁₋₆alkyl- and aryl; and
**X**_{**1**} and **X**_{**2**} are independently selected from CH, CF or N; and
**n** is 1 or 2; and
**p** is 1 or 2.

2. A compound of formula (I) according to claim 1, wherein
**X**_{**2**} represents CH or CF; and
**n** represents 1.

3. A compound of formula (Ia), 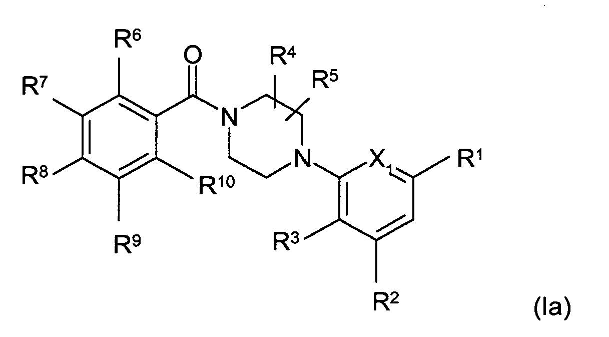 its salts or pharmaceutically acceptable derivatives thereof, wherein
**R**^{**1**} and **R**^{**2**} are independently selected from the group consisting of hydrogen, halogen and cyano or from an optionally substituted group consisting of C₁₋₆alkyl-, R^{a}O-, R^{a}R^{b}N-, C₁₋₆alkyl-C(=O)-, C₁₋₆alkyl-C(=O)-N(R^{b})-, C₁₋₆alkyl-N(R^{b})-C(=O)- and R^{a}O-C₁₋₆alkyl-; and
**R**^{**3**} is selected from the group consisting of hydrogen, halogen, cyano, C₁₋₆alkyl- and R^{a}O-, or
**R**² and **R**³ may combine to form an optionally substituted cyclic group -O-(CH₂)p-O-; and
**R**⁴ and **R**⁵ are independently selected from hydrogen and C₁₋₆alkyl-; and
**R**⁶, **R**⁷, **R**⁸, **R**⁹ and **R**¹⁰ are independently selected from the group consisting of hydrogen, halogen, nitro and cyano or from an optionally substituted group consisting of C₁₋₆alkyl-, R^{a}R^{b}N-, R^{a}O-, R^{a}-C(=O)-, R^{a}-C(=O)-N(R^{b})-, R^{a}R^{b}N-C(=O)-, R^{a}R^{b}N-SO₂-, R^{a}-SO₂-N(R^{b}), R^{a}-C(=O)-, R^{a}-SO₂-, R^{a}-SO-, R^{a}-S-, R^{a}R^{b}-C₁₋₆alkyl-, R^{a}R^{b}N-C₁₋₆alkyl-O-, R^{a}O-C₁₋₆alkyl-and aryl; and
**R**^{a} and **R**^{b} are independently selected from hydrogen, C₁₋₆alkyl-, cycloalkyl-, aryl-C₁₋₆alkyl- and aryl; and
X₁ is selected from CH, CF or N; and
**p** is 1 or 2.

4. A compound according to claim 1-3, wherein X₁ represents CH or CF.

5. A compound according to claim 1-4, wherein
**R**^{**6**}**, R**^{**7**}, **R**^{**8**}**, R**^{**9**} and **R**^{**10**} are independently selected from the group consisting of hydrogen, halogen, nitro and cyano or from an optionally substituted group consisting of C₁₋₆alkyl-, R^{a}R^{b}N-, R^{a}O-, R^{a}-C(=O)-, R^{a}R^{b}N-C₁₋₆alkyl-, R^{a}R^{b}N-C₁₋₆alkyl-O-, R^{a}O-C₁₋₆alkyl- and aryl.

6. A compound of formula (I) or (Ia) according to claim 1 - 5 as medicament.

7. A compound of formula (I) or (Ia) according to claim 1 ― 5 as antiproliferative medicament.

8. Use of a compound of formula (I) or (Ia) according to claim 1 ― 5 for the manufacture of a medicament for the treatment of a proliferative disease.

9. Use of a compound of formula (I) or (Ia) according to claim 1 ― 5 for the manufacture of a medicament for the treatment of cancer.

10. Use of a compound of formula (I) or (Ia) according to claim 1 ― 5 for the manufacture of a medicament for the treatment of conditions ameliorated by an inhibitory action on tubulin polymerization

11. Pharmaceutical composition containing as active ingredient one or more compounds of formula (I) or (Ia) according to claim 1 ― 5, or their physiologically acceptable salts in combination with a usual adjuvants and/or carrier.

12. A pharmaceutical composition comprising a compound of formula (I) 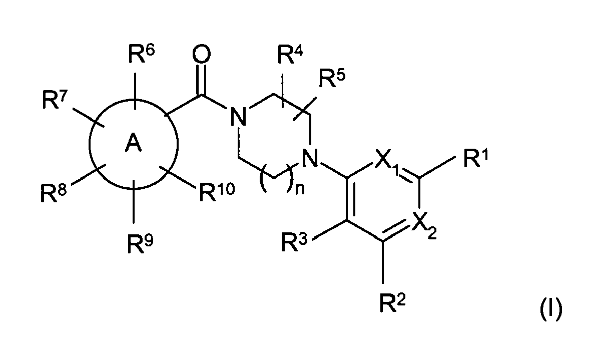 its salts or pharmaceutically acceptable derivatives thereof, wherein
A represents mono- or a bicyclic carbocyclic aryl; and
**R**¹ and **R**² are independently selected from the group consisting of hydrogen, halogen and cyano or from an optionally substituted group consisting of C₁₋₆alkyl-, R^{a}O-, R^{a} R^{b}N-, C₁₋₆alkyl-C(=O)-, C₁₋₆alkyl-C(=O)-N(R^{b})-, C₁₋₆alkyl-N(R^{b})-C(=O)- and R^{a}O-C₁₋₆alkyl-, and
**R**^{**3**} is selected from the group consisting of hydrogen, halogen, cyano, C₁₋₆alkyl- and R^{a}O-, or
**R**^{**2**} and **R**^{**3**} may combine to form an optionally substituted cyclic group -O-(CH₂)ₚ-O-; and
**R**^{**4**} and **R**^{**5**} are independently selected from hydrogen and C₁₋₆alkyl-; and
**R**^{**6**}**, R**^{**7**}**, R**^{**8**}**, R**^{**9**} and **R**^{**10**} are independently selected from the group consisting of hydrogen, halogen, nitro, cyano or from an optionally substituted group consisting of C₁₋₆alkyl-, R^{a}R^{b}N-, R^{a}O-, R^{a}-C(=O)-N(R^{b})-, R^{a}R^{b}N-C(=O)-, R^{a}R^{b}N-SO₂-, R^{a}-SO₂-N(R^{b})-, R^{a}-C(=O)-, R^{a}-SO₂₋, R^{a}-SO-, R^{a}-S-, R^{a}R^{b}N-C₁₋₆alkyl-, R^{a}R^{b}N-C₁₋₆alkyl-O-, R^{a}O-C₁₋₆alkyl-, and aryl; and
**R**^{**a**} and **R**^{**b**} are independently selected from hydrogen, C₁₋₆alkyl-, cycloalkyl-, aryl-C₁₋₆alkyl- and aryl; and
**X**_{**1**} and **X**_{**2**} are independently selected from CH, CF or N; and
**n** is 1 or 2; and
**p** is 1 or 2
or a tautomer, a pharmaceutically acceptable salt, solvate, or physiologically functional derivative thereof, and
at least one different cytostatic and/or cytotoxic active ingredient or a pharmaceutically acceptable salt, solvate, or physiologically functional derivative thereof, and
a pharmaceutically acceptable carrier or excipient.
